# EUROPÄISCHE PATENTANMELDUNG

(11) **EP 4 151 625 A1**
(43) Veröffentlichungstag der Anmeldung: **22.03.2023**
(21) Anmeldenummer: 21198127.9
(22) Anmeldetag: 21.09.2021
(51) Int. Cl.: C07C 309/24, C11D 1/00

(54) **NEUE SULFONAT-VERBINDUNGEN**

(71) Anmelder: Karl-Franzens-Universität Graz, 8010 Graz (AT); RIJKSUNIVERSITEIT GRONINGEN, 9712 CP Groningen (NL)
(72) Erfinder: Barta Weissert, Katalin, 8010 Graz (AT); Hochegger, Markus, 8010 Graz (AT); Fridrich, Bálint, 9747 AG Groningen (NL)
(74) Vertreter: Ellmeyer, Wolfgang

(57) **Zusammenfassung**

Die Erfindung betrifft neue Sulfonat-Verbindungen der Formel (I) oder (II): 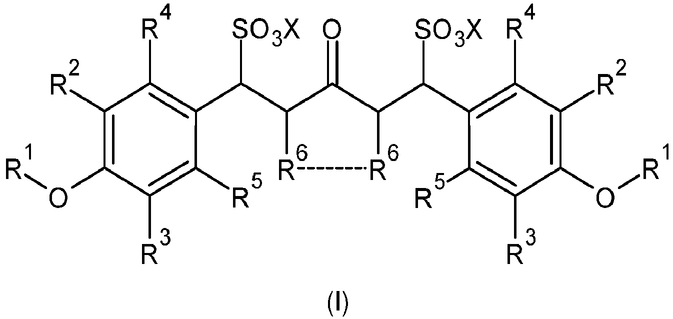 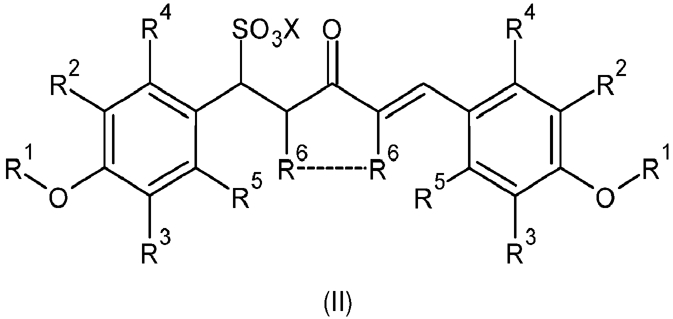 worin
die R¹ aus Kohlenwasserstoff-Resten mit 4 bis 26 C-Atomen und gegebenenfalls zumindest einem O- oder S-Atom ausgewählt sind;
R² bis R⁵ jeweils unabhängig aus Wasserstoff und Kohlenwasserstoff-Resten mit 1 bis 26 C-Atomen und gegebenenfalls zumindest einem O- oder S-Atom ausgewählt sind;
die R⁶ jeweils unabhängig aus Wasserstoff und Kohlenwasserstoff-Resten mit 1 bis 6 Kohlenstoffatomen ausgewählt sind, wobei die beiden Reste R⁶ gegebenenfalls, wie durch die gestrichelte Linie angedeutet, miteinander zu einem den Carbonyl-Kohlenstoff umfassenden fünf- oder sechsgliedrigen Ring verbunden sind; und
die X jeweils unabhängig aus ein- und mehrwertigen Kationen Xⁿ⁺, worin n ≥ 1 ist, einschließlich H⁺, ausgewählt sind, wobei in Formel (I) gegebenenfalls beide X zusammen ein mehrwertiges Kation darstellen;

sowie ein Herstellungsverfahren dafür und ihre Verwendung als Tenside.

## Beschreibung

Die vorliegende Erfindung betrifft neue Sulfonat-Verbindungen, ein Herstellungsverfahren dafür sowie deren Verwendung als Tenside.

### HINTERGRUND DER ERFINDUNG

Lignin stellt ein im Überfluss vorkommendes aromatisches Biopolymer dar, dessen Struktur hauptsächlich auf drei substituierten Phenolen, so genannten Monolignolen (p-Coumaryl-, Coniferyl- und Sinapylalkohol), basiert, die durch eine Vielzahl von unterschiedlichen C-O- und C-C-Bindungen eine amorphe 3-dimensionale Struktur ausbilden. Unterschiedliche Methoden wurden entwickelt, um den katalytischen Abbau von Lignin mittels Depolymerisation zu ermöglichen, um industriell verwertbare monomolekulare Phenol- und/oder Benzaldehyd-Derivate erhalten zu können. Neben Monomeren, können die Produkte dieser Depolymerisationsprozesse auch phenolische Di-, Tri- und Oligomere enthalten.

Erst kürzlich wurden verschiedene Methoden entwickelt, durch welche eine selektive Depolymerisation von Lignin möglich geworden ist, wobei es sich hauptsächlich um reduktive oder oxidative Reaktionsstrategien handelt. Durch Erstere werden üblicherweise Phenol-, Guajacol- oder Syringol-Derivate mit aliphatischen Resten gewonnen, die typischerweise ein bis drei Kohlenstoffatome lang und mit Alkohol-, Aldehyd-, Ester- und/oder Ketonfunktionalitäten versehen sind. Letztere liefern hingegen typischerweise aromatische Aldehyde wie Vanillin oder Syringaldehyd oder ähnlich funktionalisierte Guajacol- und Syringol-Derivate. Die Hauptprodukte solcher Depolymerisationsverfahren umfassen Guajacol und Syringol bzw. Vanillin und Syringaldehyd, die häufig jeweils einen oder mehrere Alkyl- und/oder Alkoxy-Substituenten am aromatischen Ring aufweisen.

All die genannten Ligninabbauprodukte stellen wertvolle Ressourcen auf biologischer Basis dar, aus denen in den letzten Jahren auch eine Reihe verschiedenster Produkte hergestellt wurden. Den Recherchen der Erfinder zufolge befinden sich darunter allerdings kaum Tenside, obwohl auch auf diesem Gebiet ein Bedarf an Produkten besteht, die auf der Grundlage nicht-essbarer nachwachsender Rohstoffe synthetisierbar sind. Ziel der Erfindung war vor diesem Hintergrund die Synthese neuer chemischer Verbindungen mittels Funktionalisierung dieser Produkte des Ligninabbaus sowie ähnlicher Verbindungen, vorzugsweise auf eine umweltschonende Weise, die sich insbesondere zur Verwendung als Tenside eignen.

### ZUSAMMENFASSUNG DER ERFINDUNG

Dieses Ziel erreicht die vorliegende Erfindung in einem ersten Aspekt durch Bereitstellung von neuen Sulfonat-Verbindungen der Formel (I) oder (II): 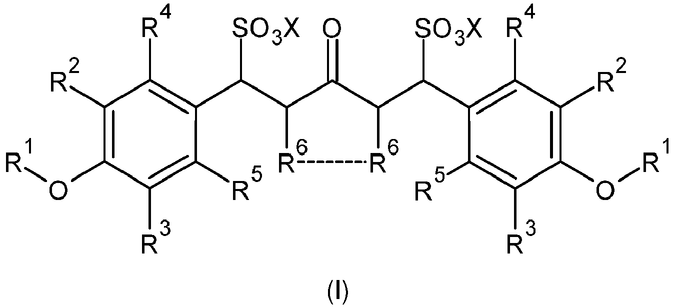 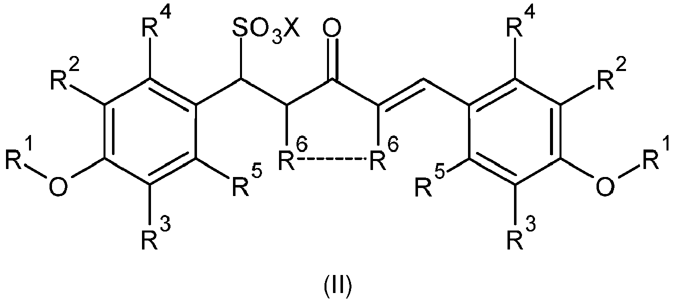 worin
die R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt sind;
R² bis R⁵ jeweils unabhängig aus Wasserstoff und linearen, verzweigten oder zyklischen Kohlenwasserstoff-Resten mit 1 bis 26 Kohlenstoffatomen, in denen gegebenenfalls zumindest ein Kohlenstoffatom durch ein Sauerstoff- oder Schwefelatom ersetzt ist, ausgewählt sind;
die R⁶ jeweils unabhängig aus Wasserstoff und gesättigten Kohlenwasserstoff-Resten mit 1 bis 6 Kohlenstoffatomen ausgewählt sind, wobei die beiden Reste R⁶ gegebenenfalls, wie durch die gestrichelte Linie angedeutet, miteinander zu einem den Carbonyl-Kohlenstoff umfassenden fünf- oder sechsgliedrigen Ring verbunden sind; und
die X jeweils unabhängig aus ein- und mehrwertigen Kationen Xⁿ⁺, worin n ≥ 1 ist, einschließlich H+, ausgewählt sind, wobei in Formel (I) gegebenenfalls beide X zusammen ein mehrwertiges Kation darstellen.

Derartige Disulfonat-Verbindungen der Formel (I) oder entsprechende Monosulfonate der Formel (II) lassen sich, wie die Erfinder festgestellt haben, auf relativ einfache sowie umweltschonende Weise aus leicht verfügbaren Lignin-Abbauprodukten herstellen und eignen sich ausgezeichnet als Tenside. Aufgrund der starken Hydrophilie der Sulfonat-Gruppierung(en) und der Hydrophobie des bzw. der Aromaten reicht sogar eine einstellige Anzahl an Kohlenstoffatomen in den Resten R¹ bis R⁵ aus, um den Verbindungen die erforderliche Amphiphilie zu verleihen.

Vorzugsweise beträgt die Anzahl an Kohlenstoffatomen in den Resten R¹ bis R⁵ jedoch zumindest 9 Kohlenstoffatome. Dies ist vor allem im Hinblick auf die Hydrophobie zu bevorzugen, wenn in den Verbindungen der Formel (I) zwei Sulfonat-Gruppierungen -SO₃X an das zentrale Pentanon gebunden sind. Aber auch der Umstand, dass zu den Hauptprodukten der Depolymerisation von Lignin, wie eingangs erwähnt, Derivate von Vanillin und Syringaldehyd zählen, die häufig noch einen oder zwei zusätzliche Niederalkyl- und/oder -alkoxy-Substituenten aufweisen, vereinfacht die Synthese von erfindungsgemäßen Sulfonat-Verbindungen mit zumindest 9 Kohlenstoffatomen in den Resten R¹ bis R⁵, da R² bis R⁴ bereits mehrere Kohlenstoffatome umfassen. Folglich braucht im erfindungsgemäßen Herstellungsverfahren bloß die freie phenolische OH-Gruppe eines solchen Vanillin- oder Syringaldehyd-Derivats mit einem leicht verfügbaren und biologisch abbaubaren Fettalkylrest verethert zu werden.

Da einerseits Fettalkohole in der Natur sowohl in gesättigter als auch in ungesättigter Form, d.h. mit einer oder mehreren C=C-Doppelbindungen vorkommen, und andererseits, wie eingangs erwähnt, die Lignin-Abbauprodukte auch mehr als einen aromatischen, aber auch nicht-aromatische Ringe (z.B. Dioxolan) als Substituenten umfassen können, sind in der Definition von R¹ bis R⁵ gemäß vorliegender Erfindung sowohl gesättigte als auch ungesättigte sowie zyklische Reste umfasst.

Dass neben den Disulfonat-Verbindungen der Formel (I) auch Monosulfonate der Formel (II) von der vorliegenden Erfindung umfasst sind, liegt an der Sulfonierung mittels Addition von Hydrogensulfit in Schritt 3) des erfindungsgemäßen Verfahrens, was in Bezug auf diesen zweiten Aspekt der Erfindung näher erläutert und durch die späteren Beispiele belegt wird.

Allgemein ist festzustellen, dass hierin unter "Sulfonat" nicht nur Salze der Sulfonsäure-Gruppe(n), sondern jeweils auch die freie Säure zu verstehen ist, sofern der Kontext nichts anderes erfordert. Dies wird auch durch die obige Definition des Gegenions X belegt, in die ausdrücklich auch H⁺ inkludiert ist. Das liegt daran, dass bei der erfindungsgemäßen Verwendung der neuen Verbindungen als Tenside gemäß dem dritten Aspekt auch die jeweiligen freien Säuren in wässriger Umgebung ionisiert werden und dadurch *in situ* Sulfonat-Gruppen ausbilden.

Die untere und obere Grenze für die Anzahl der Kohlenstoffatome in den Resten R¹ bis R⁵ bezieht sich auf die bevorzugte Verwendung von Fettalkylresten zur Veretherung von freien phenolischen OH-Gruppen in den Ausgangsprodukten, für deren Kettenlänge in der Literatur zwischen 4 bis 6 als Untergrenze und zwischen 22 und 26 als Obergrenze angegeben werden. Erfindungsgemäß bevorzugt wird eine maximale Länge von 18 Kohlenstoffatomen für die im Syntheseverfahren mittels Veretherung eingeführten Fettalkylreste bzw. von 4 Kohlenstoffatomen für die bereits im Ausgangsmaterial an den Aromaten gebundenen Alkyl- bzw. Alkoxy- oder gegebenenfalls auch Alkylthio-Resten, was insbesondere für die Reste R² und R³ in o-Stellung zur phenolischen OH-Gruppe gilt.

Die Option, dass manche Kohlenstoffatome durch Sauerstoff oder Schwefel ersetzt sein können, bezieht sich ebenfalls vor allem auf das Substitutionsmuster der vorzugsweise durch Lignin-Depolymerisation erhaltenen Ausgangsverbindungen, die, wie eingangs erwähnt, verschiedene Sauerstoff-hältige Funktionalitäten, mitunter aber auch Schwefel-Analoga davon aufweisen können. Andere Heteroatome, wie z.B. Halogene oder Stickstoff, kommen in solchen Verbindungen hingegen kaum vor. Für die Zwecke der vorliegenden Erfindung brauchen daher andere Heteroatome als Sauerstoff und Schwefel wohl nicht in Betracht gezogen zu werden.

In manchen bevorzugten Ausführungsformen der Erfindung ist daher R¹ C₆-C₂₂-Alkyl, noch bevorzugter C₈-C₁₈-Alkyl. Alternativ oder zusätzlich dazu sind in manchen bevorzugten Ausführungsformen R² und R³ aus Wasserstoff, C₁-C₂₂-Alkyl und C₁-C₂₂-Alkoxy, noch bevorzugter aus Wasserstoff, C₁-C₄-Alkyl und C₁-C₄-Alkoxy ausgewählt, wobei z.B. einer von R² und R³ Wasserstoff ist und der andere Methoxy ist oder beide Reste Methoxy sind. Alternativ oder zusätzlich dazu sind in manchen bevorzugten Ausführungsformen R⁴ und R⁵ aus Wasserstoff, Methyl und Methoxy ausgewählt. Alternativ oder zusätzlich dazu sind in manchen bevorzugten Ausführungsformen die R⁶ aus Wasserstoff, Methyl und Ethyl ausgewählt sind oder miteinander zu einem den Carbonyl-Kohlenstoff umfassenden fünf- oder sechsgliedrigen Ring verbunden. Alternativ oder zusätzlich dazu stehen in manchen bevorzugten Ausführungsformen die X jeweils für H⁺, Na⁺, K⁺, NH₄⁺ oder ein organisches Ammoniumion.

In besonders bevorzugten Ausführungsformen stehen herstellungsbedingt jeweils beide der Reste R¹ bis R⁵ für denselben Rest, d.h. beide Reste R¹ für denselben C₈-C₁₈-Alkylrest, beide Reste R² und beide Reste R³ für Wasserstoff oder jeweils denselben C₁-C₄-Alkyl- oder denselben C₁-C₄-Alkoxy-Rest sowie beide Reste R⁴ und beide Reste R⁵ für entweder Wasserstoff, Methyl oder Methoxy. Darüber hinaus stehen in den Disulfonat-Verbindungen der Formel (I) auch beide X für dasselbe Gegenion, d.h. entweder stehen beide für dasselbe einwertige Kation X⁺, oder beide X zusammen sind Teil desselben mehrwertigen Kations Xⁿ⁺, das in solchen Fällen insbesondere ein zweiwertiges Kation X²⁺, wie z.B. Ca²⁺ oder Mg²⁺, ist. In diesen Ausführungsformen ist die neue Disulfonat-Verbindung der Formel (I) um eine durch die zentrale Ketogruppe verlaufende (d.h. in der Formelzeichnung: vertikale) Achse spiegelsymmetrisch.

In manchen besonders bevorzugten Ausführungsformen gilt daher, dass:
die R¹ beide für denselben C₈-C₁₈-Alkylrest stehen;
beide R² und beide R³ jeweils Wasserstoff oder Methoxy sind;
beide R⁴ und beide R⁵ jeweils Wasserstoff sind;
die R⁶ beide Wasserstoff oder Methyl sind oder miteinander zu einem Ethylen- oder Propylenrest verbunden sind und somit einen den Carbonyl-Kohlenstoff umfassenden fünf- oder sechsgliedrigen Ring bilden; und
die X jeweils für H⁺, Na⁺, NH₄⁺ oder ein organisches Ammoniumion stehen, das insbesondere (2-Hydroxyethyl)trimethylammonium (Cholin) oder Triethanolammonium ist.

Insbesondere ist die Sulfonat-Verbindung gemäß dem ersten Aspekt der vorliegenden Erfindung aus den folgenden Verbindungen ausgewählt:
1,5-Bis(3-methoxy-4-octyloxyphenyl)-3-oxo-1,5-pentandisulfonsäurediammoniumsalz (1) 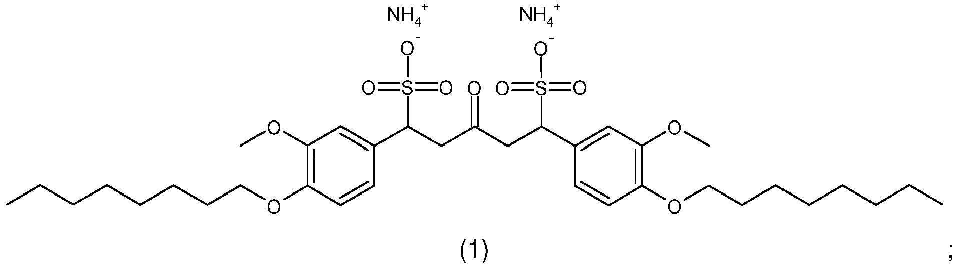
1,5-Bis(4-dodecyloxy-3-methoxyphenyl)-3-oxo-1,5-pentandisulfonsäure-diammoniumsalz (2) 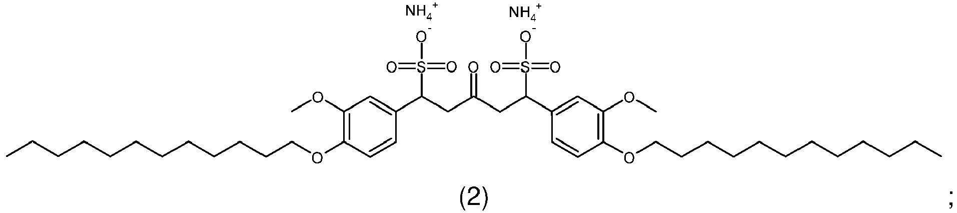
1,5-Bis(3-methoxy-4-tetradecyloxyphenyl)-3-oxo-1,5-pentandisulfonsäure-diammoniumsalz (3) 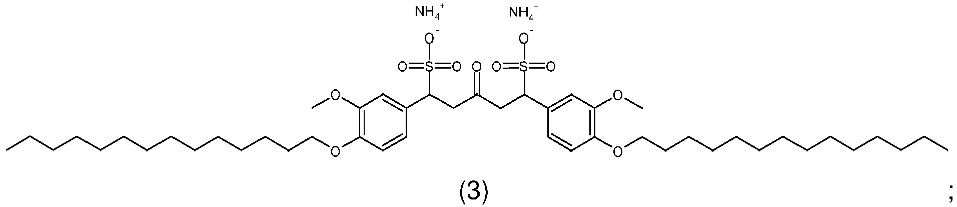
1,5-Bis(4-hexadecyloxy-3-methoxyphenyl)-3-oxo-1,5-pentandisulfonsäure-diammoniumsalz (4) 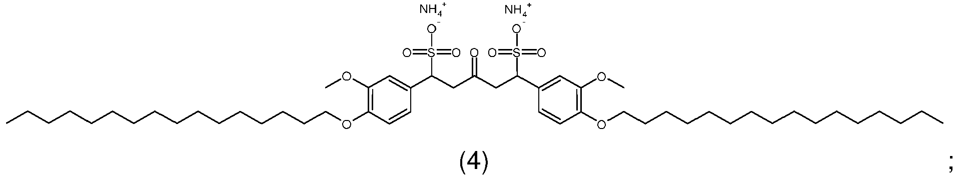
1,5-Bis(3-methoxy-4-octadecyloxyphenyl)-3-oxo-1,5-pentandisulfonsäure-diammoniumsalz (5) 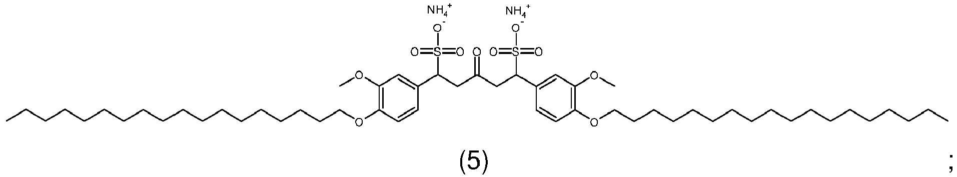
1,5-Bis(4-dodecyloxy-3-methoxyphenyl)-3-oxo-1,5-pentandisulfonsäure-dinatriumsalz (6) 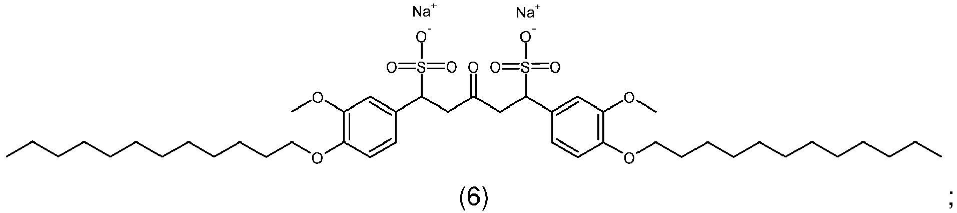
1,5-Bis(3-methoxy-4-tetradecyloxyphenyl)-3-oxo-1,5-pentandisulfonsäure-dinatriumsalz (7) 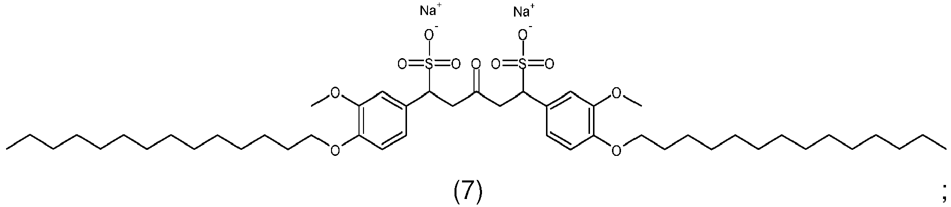
1,5-Bis(4-hexadecyloxy-3-methoxyphenyl)-3-oxo-1,5-pentandisulfonsäure-dinatriumsalz (8) 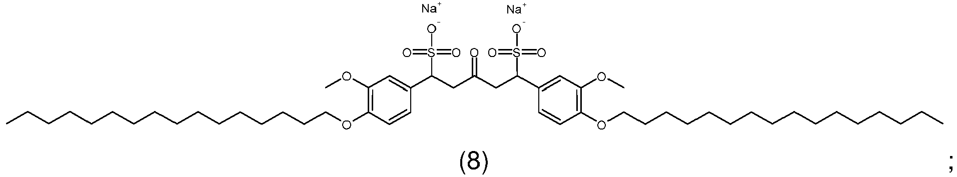
1,5-Bis(3-methoxy-4-octadecyloxyphenyl)-3-oxo-1,5-pentandisulfonsäure-dinatriumsalz (9) 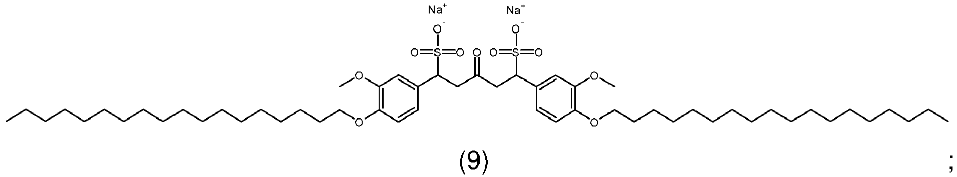
1,5-Bis(4-hexadecyloxy-3-methoxyphenyl)-3-oxo-1,5-pentandisulfonsäure-dikaliumsalz (10) 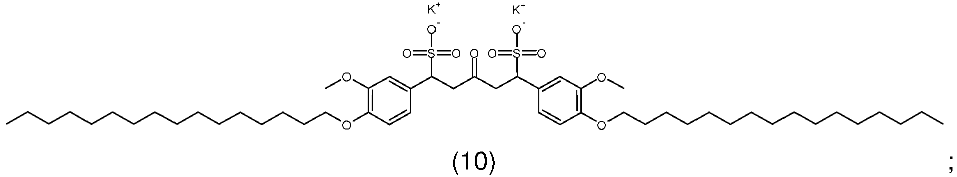
1,5-Bis(4-dodecyloxy-3-methoxyphenyl)-3-oxo-1,5-pentandisulfonsäure-dicholinsalz (11) 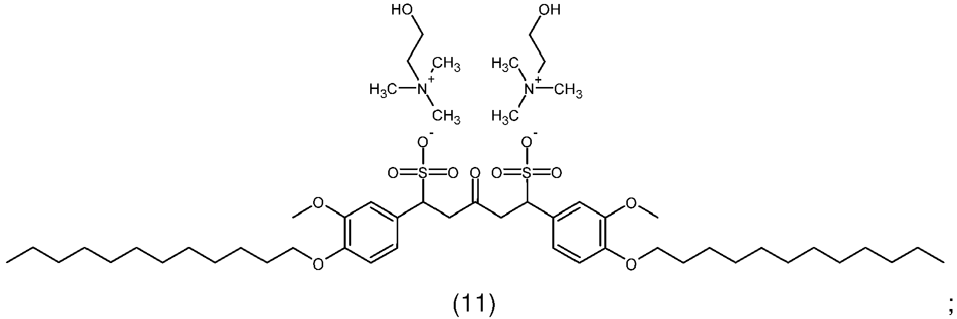
1,5-Bis(4-dodecyloxy-3-methoxyphenyl)-3-oxo-1,5-pentandisulfonsäure-bis(triethanolammonium)salz (12) 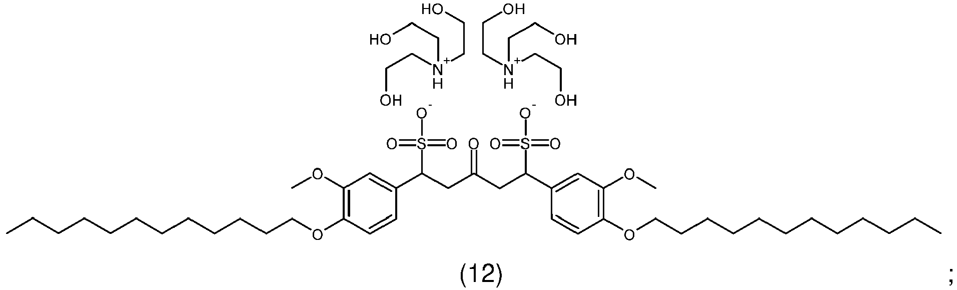
1,1'-(2-Oxocyclopentan-1,3-diyl)-bis[(3-methoxy-4-octyloxyphenyl)-methansulfonsäure-ammoniumsalz] (13)

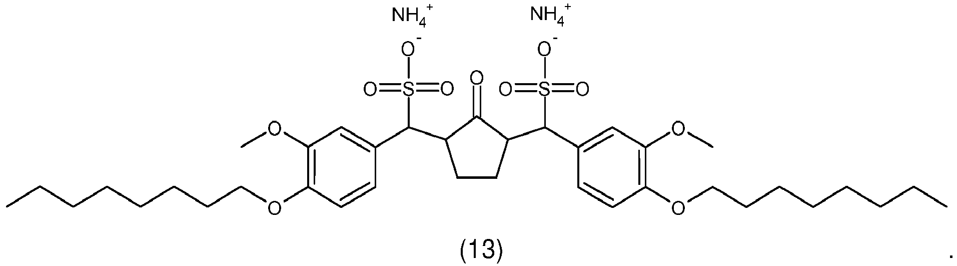

In einem zweiten Aspekt betrifft die vorliegende Erfindung ein Verfahren zur Herstellung einer Sulfonat-Verbindung gemäß dem ersten Aspekt, das die folgenden Schritte umfasst:
1) das Umsetzen eines 4-Hydroxybenzaldehyd-Derivats der nachstehenden Formel (III): 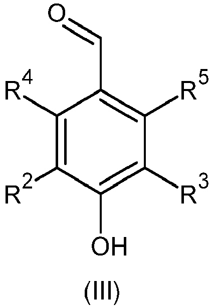 worin
   R² bis R⁵ jeweils unabhängig aus Wasserstoff und linearen, verzweigten oder zyklischen Kohlenwasserstoff-Resten mit 1 bis 26 Kohlenstoffatomen, in denen gegebenenfalls zumindest ein Kohlenstoffatom durch ein Sauerstoff- oder Schwefelatom ersetzt ist, ausgewählt sind;
   mit einer Verbindung der Formel R¹-Y, worin R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt ist und Y für eine aus Halogeniden und Sulfonaten ausgewählte Abgangsgruppe steht, mittels einer Veretherungsreaktion nach Williamson in Gegenwart einer Base in einem organischen Lösungsmittel, wodurch ein entsprechender Ether der Formel (IV) erhalten wird: 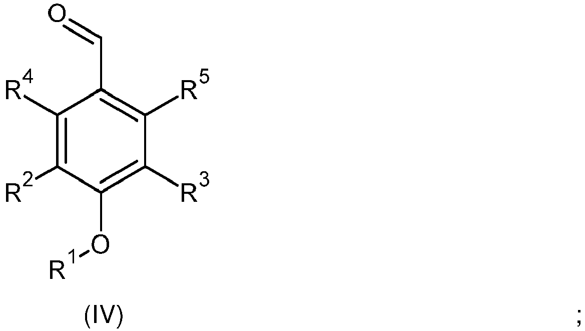
2) das Umsetzen des Ethers der Formel (IV) mit einem halben Äquivalent von Aceton oder einem Aceton-Derivat der Formel (V) 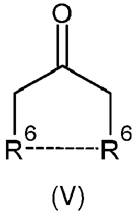 worin die R⁶ jeweils unabhängig aus Wasserstoff und gesättigten Kohlenwasserstoff-Resten mit 1 bis 6 Kohlenstoffatomen ausgewählt sind, wobei die beiden Reste R⁶ gegebenenfalls, wie durch die gestrichelte Linie angedeutet, miteinander zu einem den Carbonyl-Kohlenstoff umfassenden fünf- oder sechsgliedrigen Ring verbunden sind, mittels einer doppelten gekreuzten Aldol-Kondensationsreaktion nach Claisen-Schmidt unter Säure- oder Basenkatalyse in einem organischen Lösungsmittel, wodurch ein entsprechendes ungesättigtes Keton der Formel (VI) erhalten wird: 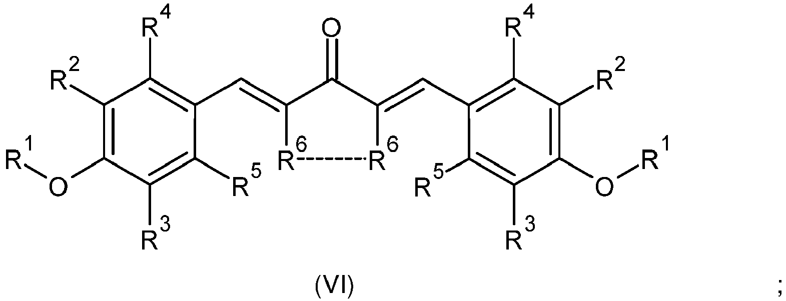
3) das Umsetzen des Ketons der Formel (VI) mit einem Sulfonierungsmittel in einem alkoholischen Lösungsmittel zur Addition von einem oder zwei Äquivalenten Hydrogensulfit an die Doppelbindung(en) des Ketons der Formel (VI), gegebenenfalls gefolgt von einem lonenaustausch des so erhaltenen Mono- oder Disulfonats sowie Einführung von vorbestimmten Gegenionen Xⁿ⁺, worin n ≥ 1 ist, um die Sulfonat-Verbindung der Formel (I) oder (II) zu erhalten.

Auf diese Weise ist es gemäß vorliegender Erfindung möglich, durch ein vergleichsweise einfaches und kostengünstiges Verfahren, das eine Abfolge an sich bekannter Einzelreaktionen umfasst, aus Hydroxybenzaldehyd-Derivaten der Formel (III) die neuen Sulfonat-Verbindungen zu synthetisieren. In bevorzugten Ausführungsformen handelt es sich bei den Ausgangsverbindungen um leicht verfügbare Produkte der Lignin-Depolymerisation, wie z.B. gegebenenfalls substituiertes Vanillin oder Syringaldehyd, und das Verfahren wird auf möglichst umweltschonende Weise durchgeführt.

In manchen bevorzugten Ausführungsformen des Verfahrens der vorliegenden Erfindung wird in Schritt 1) als Abgangsgruppe Y das Chlorid oder Bromid, insbesondere das Bromid, eingesetzt; und/oder wird als Base K₂CO₃, insbesondere zwei Äquivalente K₂CO₃, eingesetzt; und/oder wird Acetonitril als organisches Lösungsmittel, insbesondere bei Rückflusstemperatur, eingesetzt.

Als Abgangsgruppe Y kann beispielsweise auch ein Sulfonat, wie z.B. Mesylat oder Tosylat, eingesetzt werden, allerdings wäre der Einsatz langkettiger Fettalkoholsulfonate unökonomisch, da diese ja selbst bereits Tenside sind, weswegen das Chlorid oder Bromid, insbesondere das Bromid, zu bevorzugen ist. Als Lösungsmittel hat sich, nach einer Reihe von Versuchen mit anderen polaren aprotischen Lösungsmitteln, wie z.B. Aceton, Diethylether und DMF, Acetonitril, insbesondere unter Rückfluss, bewährt, da es die besten Umsätze ergeben und die anschließende Reinigung des Produkts vereinfacht hat. Aus letzterem Grund wird das Hydroxybenzaldehyd-Derivat der Formel (III) auch vorzugsweise in leichtem Überschuss gegenüber der Verbindung der Formel R¹-Y eingesetzt, z.B. in einem Überschuss von 10-15 Mol-%. In Kombination mit der Verwendung von K₂CO₃ als Base bietet dies den zusätzlichen Vorteil, dass das als Nebenprodukt gebildete KBr gewonnen und gewissermaßen rezykliert, d.h. zur Synthese weiterer gewünschter Verbindungen der Formel R¹-Br eingesetzt werden kann. Da es sich dabei vorzugsweise um Fettalkylbromide handelt, können diese mit dem KBr einfach aus Fettalkoholen hergestellt werden.

In manchen bevorzugten Ausführungsformen des Verfahrens der vorliegenden Erfindung wird in Schritt 2) Lithiumhydroxid-monohydrat LiOH.H₂O als basischer Katalysator, insbesondere in einer Menge von 1-10 Mol-%, eingesetzt; und/oder wird als organisches Lösungsmittel ein niederer Alkohol, ein Ether oder ein Gemisch davon, noch bevorzugter Isopropanol, eingesetzt, wobei sich insbesondere eine Reaktionstemperatur von 40-50 °C bewährt hat.

Als basischer Katalysator können zwar auch andere Basen, wie z.B. NaOH oder KOH, eingesetzt werden, oder die Reaktion kann unter Phasentransfer-Katalyse, z.B. unter Verwendung von Tetra-n-butylammoniumbromid (TBAB) als Katalysator, durchgeführt werden. Allerdings haben die Erfinder mit Lithiumhydroxid-monohydrat LiOH.H₂O die besten Ergebnisse in Bezug auf Umsatz und Reaktionsdauer erzielt. Ähnliches gilt für das bevorzugte Lösungsmittel Isopropanol, anstelle dessen auch Ether, andere Alkohole wie MeOH oder EtOH, oder Gemische eines Alkohols mit Wasser oder Diethylether einsetzbar sind. Bei Verwendung von Isopropanol brauchen die Reaktionsgemische gar nicht auf dessen Rückflusstemperatur (82,5 °C) erhitzt zu werden, um in kurzer Zeit hohe Umsätze zu erzielen.

In manchen bevorzugten Ausführungsformen des Verfahrens der vorliegenden Erfindung wird weiters in Schritt 3)
als Sulfonierungsmittel ein Hydrogensulfit oder Disulfit, noch bevorzugter Natriumhydrogensulfit Na₂S₂O₅, Calciumhydrogensulfit Ca(HSO₃)₂, Ammoniumhydrogensulfit NH₄HSO₃ oder Trimethylammoniumsulfit [(CH₃)₃N]₂SO₃, eingesetzt; und/oder
ein Gemisch aus einem niederen Alkohol und Wasser, noch bevorzugter wässriges Methanol oder Isopropanol, als alkoholisches Lösungsmittel eingesetzt; und/oder
als Katalysator ein Amin, noch bevorzugter Triethylamin, Triethanolamin oder Cholinhydroxid, eingesetzt.

In besonders bevorzugten Ausführungsformen werden das Hydrogensulfit oder Disulfit in einer Menge von drei Äquivalenten Hydrogensulfit und der Amin-Katalysator in einer Menge von zumindest 20 Mol-%, jeweils bezogen auf das Keton der Formel (VI), eingesetzt und wird als Lösungsmittel wässriges Isopropanol unter Rückfluss eingesetzt, um eine Disulfonat-Verbindung der Formel (I) zu erhalten. Zur Synthese einer Monosulfonat-Verbindung der Formel (II) wird das das Hydrogensulfit oder Disulfit hingegen nur in einer Menge von einem Äquivalent Hydrogensulfit eingesetzt, um nur eine Sulfonsäuregruppe an eine der beiden Doppelbindungen des Ketons der Formel (VI) zu addieren. Darüber hinaus ist es durch Variation der Hydrogensulfit-Äquivalente auch möglich, Gemische aus einem Disulfonat der Formel (I) und einem Monosulfonat der Formel (II) herzustellen, von denen entweder die beiden erhaltenen Produkte isoliert werden können oder auch das Gemisch als solches, gegebenenfalls nach einem anschließenden lonenaustausch, als Tensid eingesetzt werden kann.

Sofern nicht bereits im Verlauf der Sulfonierung das jeweils gewünschte Gegenion in die Sulfonat-Verbindung der Formel (I) oder (II) eingeführt wurde, z.B. durch Verwendung des entsprechenden Amins als Katalysator, wird das in Schritt 3) das erhaltene Sulfonat-Addukt vorzugsweise zunächst einem lonenaustausch unter Verwendung eines geeigneten sauren Ionenaustauscherharzes und Elution mit Wasser zur Überführung der Sulfonat-Gruppe(n) in (eine) freie Sulfonsäure-Gruppe(n) unterzogen, die gegebenenfalls mit einer wässrigen Lösung der Hydroxide vorbestimmter Gegenionen Xⁿ⁺ neutralisiert wird, um die jeweils gewünschte Sulfonat-Verbindung der Formel (I) oder (II) zu erhalten.

In besonders bevorzugten Ausführungsformen des erfindungsgemäßen Verfahrens werden die Schritte 2) und 3) als Eintopf-Reaktion ohne Isolierung des Ketons der Formel (VI) durchgeführt, indem beide Reaktionen in demselben niederen Alkohol als Lösungsmittel durchgeführt werden, wobei nach Beendigung der doppelten gekreuzten Aldol-Kondensationsreaktion, z.B. unter Verwendung von 10 Mol-% LiOH.H₂O als basischer Katalysator in alkoholischem Lösungsmittel, insbesondere Isopropanol, für eine Reaktionszeit von 12 h bei 45 °C, einfach das Sulfonierungsmittel in Form einer wässrigen Lösung, z.B. von NaHSO₃, der basische Katalysator, z.B. ein Amin wie Triethylamin oder bereits das als Gegenion gewünschte Amin, sowie gegebenenfalls eine zusätzliche Menge desselben alkoholischen Lösungsmittels, zugesetzt werden und das Gemisch besonders bevorzugt rückflusserhitzt wird, z.B. für eine Reaktionszeit von 12-14 h. Dabei kann der Reaktionsfortschritt beispielsweise anhand des allmählichen Verschwindens der durch die konjugierte aromatische Struktur des Ketons der Formel (VI) bedingten Färbung, z.B. einer intensiven Gelbfärbung, des Reaktionsgemischs verfolgt werden.

Die Aufarbeitung der die erfindungsgemäßen Sulfonat-Verbindungen der Formel (I) oder (II) enthaltenden Reaktionsgemische erfolgt in bevorzugten Ausführungsformen zunächst durch Einleiten von O₂ bei Raumtemperatur, um überschüssiges Sulfit zum Sulfat zu oxidieren, und anschließende Zugabe von n-Pentan sowie Abdestillieren des ternären Azeotrops aus Wasser, dem niederen Alkohol, insbesondere Isopropanol, und Pentan, bei einer mittleren Temperatur von z.B. 40-60 °C, vorzugsweise unter Verwendung eines Wasserabscheiders nach Dean-Stark, wodurch der Großteil des Wassers und des niederen Alkohols entfernt wird. Durch darauffolgende Trocknung am Rotationsverdampfer im Vakuum wird ein zumeist leicht gelblicher fester Rückstand erhalten. Dieser wird in der Folge vorzugsweise durch Extraktion mit einem organischen Lösungsmittel, wie z.B. Aceton, CHCl3, CH₂Cl₂ oder Et₂O, beispielsweise unter Verwendung eines Soxhlet-Extraktionsaufsatzes, bis zur Entfärbung gereinigt.

Und in einem dritten Aspekt betrifft die vorliegende Erfindung die Verwendung der neuen Sulfonat-Verbindungen der Formel (I) oder (II), in denen die Gesamtanzahl der Kohlenstoffatome der Reste R¹ bis R⁵ zumindest 9 betragen sollte, als Tenside.

### BEISPIELE

Die vorliegende Erfindung wird nachstehend anhand von Beispielen näher beschrieben, die jedoch nicht als Einschränkung des Schutzumfangs zu verstehen sind. Zur Illustration wurde als repräsentative Modellverbindung für die als Ausgangssubstanzen im erfindungsgemäßen Verfahren bevorzugten häufigen Lignin-Depolymerisationsprodukte Vanillin eingesetzt und zu den erfindungsgemäßen Sulfonat-Verbindungen umgesetzt. Weitere Beispiele unter Verwendung seines Methoxy-Derivats Syringaldehyd, einem weiteren typischen aromatischen Aldehyd als Abbauprodukt von Lignin, sind derzeit Gegenstand weiterer Experimente der Erfinder.

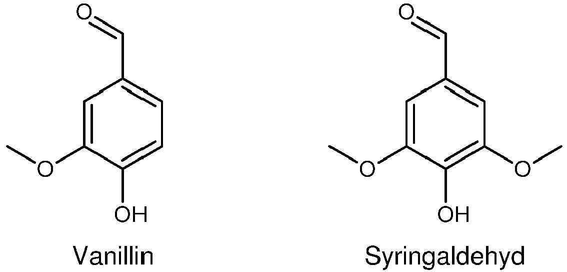

Gemäß nachstehendem Reaktionsschema A wurde das Vanillin (III) zunächst mit einer Reihe verschiedener Fettalkylhalogenide R¹-Y verethert, wonach die so erhaltenen Ether der Formel (IV) jeweils mit einem halben Äquivalent Aceton oder Cyclopentanon (wie durch die gestrichelten Bindungen angedeutet) einer doppelten gekreuzten Aldol-Kondensationsreaktion zum Erhalt doppelt ungesättigter Ketone der Formel (VI) unterzogen wurden, an die abschließend Hydrogensulfit addiert wurde, um die entsprechende erfindungsgemäße Sulfonat-Verbindung zu erhalten. In letzten Schritt wurden bisher ausschließlich zwei Äquivalente Hydrogensulfit an beide Doppelbindungen des Ketons addiert, um Disulfonate der Formel (I) zu erhalten. Für den Fachmann ist es jedoch selbstverständlich, dass auf analoge Weise, nur unter Verwendung einer geringeren Menge an Sulfonierungsmittel auch entsprechende Monosulfonate der Formel (II) oder Gemische von beiden erhalten werden können, wie dies zuvor bereits festgestellt wurde.

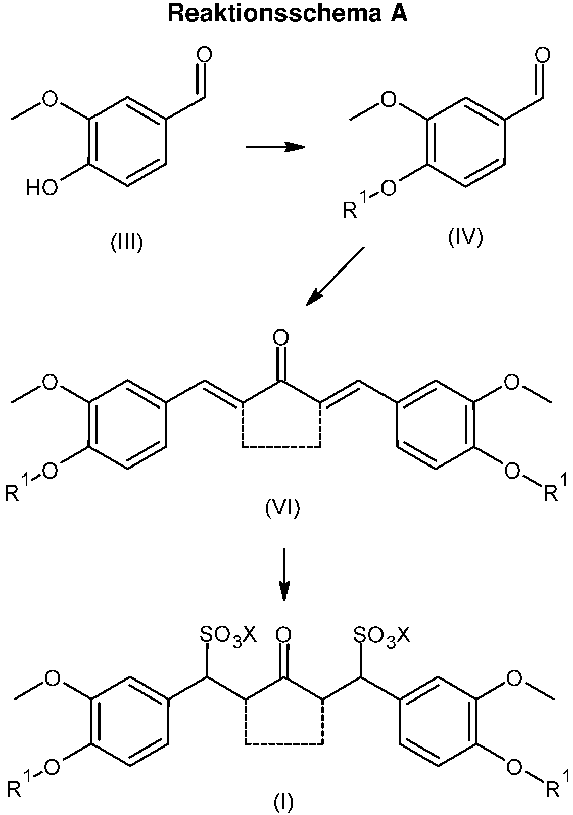

### Beispiel 1

### Herstellung von 1,5-Bis(3-methoxy-4-octyloxyphenyl)-3-oxo-1,5-pentandisulfonsäure-diammoniumsalz (1)

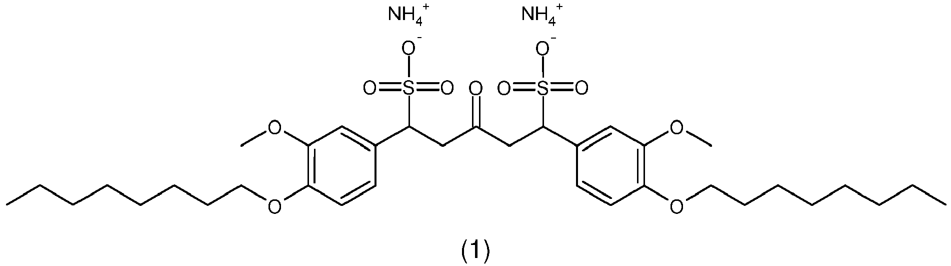

Stufe 1:
Vanillin (6,39 g, 42 mmol) wurde mit 1-Bromoctan (6,76 g, 35 mm) und ofengetrocknetem K₂CO₃ in 100 ml Acetonitril unter Stickstoffatmosphäre 48 h lang rückflusserhitzt.
Anschließend wurde das Lösungsmittel am Rotationsverdampfer entfernt, und der erhaltene Rückstand wurde in 200 ml eines 1:1-Gemischs aus Diethylether (Et₂O) und Petrolether (Kp.: 40-60°C) erneut gelöst und mit 50 ml 0,5 M NaOH und danach mit 50 ml Wasser gewaschen. Die organische Phase wurde über NaSO₄ getrocknet, wonach das Lösungsmittel im Vakuum abdestilliert und der Rückstand im Vakuumexsikkator vollständig getrocknet, wodurch das alkylierte Vanillin, 3-Methoxy-4-octyloxybenzaldehyd, als weißes Pulver erhalten wurde (Ausbeute: 9,13 g; 98,7 % d.Th.).

Stufe 2:
4-Octyloxy-3-methoxybenzaldehyd (1,85 g, 7 mmol) wurde in 14 ml Isopropanol bei 45 °C 12 h lang mit Aceton (0,20 g, 3,5 mmol) in Gegenwart von Lithiumhydroxid-monohydrat LiOH.H₂O (14,7 g, 0,35 mmol) als Katalysator umgesetzt. Anschließend wurde der feste Niederschlag abzentrifugiert, mit 3x mit 6 ml MeOH gewaschen und im Vakuum getrocknet, wodurch das doppelte Addukt, 1,5-Bis(3-methoxy-4-octyloxyphenyl)penta-1,4-dien-3-on, als gelbes Pulver erhalten wurde (Ausbeute: 1,65 g; 85,4 % d.Th.).

Stufe 3:
*Variante 3.1*
   1,5-Bis(3-methoxy-4-octyloxyphenyl)penta-1,4-dien-3-on (1,1 g, 2 mmol) in 22 ml Isopropanol wurde mit 6 ml einer 1 M wässrigen Lösung von Natriumhydrogensulfit, NaHSO₃, in Gegenwart von Triethylamin (0,04 g, 0,4 mmol) unter Rückfluss 14 h lang umgesetzt. Anschließend wurde der feste Niederschlag abzentrifugiert und mit MeOH gewaschen. Die vereinigten organischen Phasen wurden durch einen 0,2-µm-Spritzenfilter filtriert, im Vakuum eingeengt und danach durch ein Bett aus frisch aktiviertem saurem lonenaustauscherharz (DOWEX^{®} 50WX8-100) geleitet und 3x mit 50 ml Wasser gewaschen, wonach konzentrierte NH₄OH-Lösung in leichtem Überschuss zugesetzt wurde, um das Ammoniumsalz herzustellen, das abfiltriert und im Vakuumexsikkator vollständig getrocknet wurde, wodurch die Titelverbindung (1) als weißer Feststoff erhalten wurde (Ausbeute: 1,34 g; 89,2 % d.Th.).
*Variante 3.2*
   1,5-Bis(3-methoxy-4-octyloxyphenyl)penta-1,4-dien-3-on (28 mg, 0,05 mmol) wurde in einem Mikrowellenröhrchen unter Verwendung eines Teflon-beschichteten Rührstäbchens mit 0,1 ml einer 1,5 M wässrigen Lösung von Natriumhydrogensulfit, NaHSO₃, und Triethylamin (1 mg, 0,01 mmol) vermischt, dicht verschlossen und in einem Mikrowellenreaktor 45 min lang auf 140 °C erhitzt. Das Reaktionsgemisch wurde danach im Vakuum getrocknet, und die rohe Probe wurde zur Analyse mittels NMR-Spektroskopie in MeOD gelöst.

**¹H-NMR:** δ_{H} (700 MHz, MeOD) 6,97 (d, 1H, (C6,C12)), 6,89 (s, 1H, (C6^{m}, C12^{m})), 6,78 (m, 2H (C3, C4, C8, C9)), 6,70 (m, 2H, (C3^{m}, C4^{m}, C8^{m}, C9^{m})), 4,33 (m, 1H, (C13, C18)), 4,26 (m, 1H, (C13^{m}, C18^{m})), 3,96 (m, 4H, (C33, C41)), 3,81 (s, 3H, (C20, C22)), 3,76 (s, 3H, (C20^{m}, C22^{m})), 3,34-3,25 (m, 4H, (C14, C16)), 1,78 (m, 4H, (C34, C42)), 1,48 (m, 4H, (C35, C43)), 1,35 (m, 17H (C36, C37, C38, C39, C44, C45, C46, C47)), 0,92 (t, 6H (C40, C48)). ¹³**C-NMR:** δ_{C} (176 MHz, MeOD) 207,4 (C15^{m}), 206,8 (C15), 150,3 (C1, C11), 150,1 (C1^{m}, C11^{m}), 149,4 (C2, C10), 149,3 (C2^{m}, C10^{m}), 130,5 (C5, C7), 130,3 (C5^{m}, C7^{m}), 123,0 (C4, C8), 122,5 (C4^{m}, C8^{m}), 114,8 (C6, C12), 114,1 (C3, C9), 113,9 (C3^{m}, C9^{m}), 70,2 (C33, C41), 70,1 (C33^{m}, C41^{m}), 62,6 (C13^{m}, C18^{m}), 62,3 (C13, C18), 56,4 (C20, C22), 56,3 (C20^{m}, C22^{m}), 46,6 (C14^{m}, C16^{m}), 46,0 (C14, C16), 33,1 (C34^{m}, C42^{m}), 33,0 (C34, C42), 30,6 (C35^{m}, C43^{m}), 30,5 (C35, C43), 30,5 (C36, C44), 30,5 (C36^{m}, C44^{m}), 30,5 (C37^{m}, C45^{m}), 30,4 (C37, C45), 27,2 (C38^{m}, C46^{m}), 27,2 (C38, C46), 23,8 (C39^{m}, C47^{m}), 23,7 (C39, C47), 14,5 (C40, C48). **Elementar-analyse:** ber.: C, 56,13; H, 8,07; N, 3,74; S, 8,56; gef.: C, 55,13; H, 8,33; N, 3,63; S, 8,36. **HRMS:** (ESI⁺, m/z) ber. für C₃₅H₆₁N₂O₁₁S₂ [M+H]⁺: 749,37168; gef.: 749,370948.

### Beispiel 2

### Herstellung von 1,5-Bis(4-dodecyloxy-3-methoxyphenyl)-3-oxo-1,5-pentandisulfonsäure-diammoniumsalz (2)

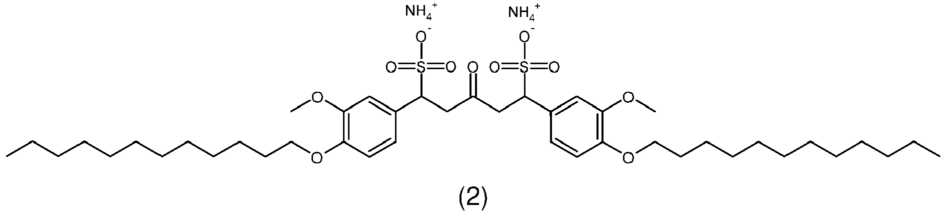

Stufe 1:
Die Reaktion erfolgte analog zu jener in Beispiel 1, außer dass 1-Bromdodecan anstelle von 1-Bromoctan eingesetzt wurde, wobei 4-Dodecyloxy-3-methoxybenzaldehyd als cremefarbenes Pulver erhalten wurde (Ausbeute: 10,85 g; 96,8 % d.Th.).

Stufe 2:
Die Reaktion erfolgte analog zu jener in Beispiel 1, wobei 1,5-Bis(4-dodecyloxy-3-methoxyphenyl)penta-1,4-dien-3-on als gelbes Pulver erhalten wurde (Ausbeute: 20,0 g; 86,0 % d.Th.).

Stufe 3:
Die Reaktion erfolgte analog zu jener in Beispiel 1, außer dass nur 1 mmol 1,5-Bis(4-dodecyloxy-3-methoxyphenyl)penta-1,4-dien-3-on eingesetzt wurde, wobei die Titelverbindung (2) als cremefarbenes Pulver erhalten wurde (Ausbeute: 0,75 g; 87,0 % d.Th.).

**¹H-NMR:** δ_{H} (300 MHz, MeOD) 6,94 (1 H, s, (C6, C12)), 6,85 (1 H, s, (C6^{m}, C12^{m})), 6,73 (2 H, d, *J* 1,1, (C3, C4, C8, C9)), 6,69-6,63 (2 H, m, (C3^{m}, C4^{m}, C8^{m}, C9^{m})), 4,34-4,27 (1 H, m, (C13, C18)), 4,26-4,17 (1 H, m, (C13^{m}, C18^{m})), 3,99-3,87 (4 H, m, (C14, C16)), 3,78 (2 H, s, (C20, C22)), 3,73 (4 H, s (C20^{m}, C22^{m})), 3,36-3,12 (4 H, m (C14, C16)), 1,84-1,67 (4 H, m (C34, C46)), 1,52-1,41 (4 H, m (C35, C47)), 1,41-1,24 (33 H, m), 0,89 (6 H, t, *J* 6,8 (C44, C56)). ¹³**C-NMR:** δ_{C} (75 MHz, MeOD) 207,4 (C15^{m}), 206,8 (C15), 150,2 (C1, C11), 150,1 (C1^{m}, C11^{m}), 149,4 (C2, C10), 149,3 (C2^{m}, C10^{m}), 130,4 (C5, C7), 130,3( C5^{m}, C7^{m}), 123,0 (C4, C8), 122,4 (C4^{m}, C8^{m})), 114,8 (C6, C12), 114,0 (C3, C9), 113,9 (C3^{m}, C9^{m}), 70,2 (C33, C45), 70,1 (C33^{m}, C45^{m}), 62,6 (C13^{m}, C18^{m}), 62,3 (C13, C18), 56,4 (C20, C22), 56,3 (C20^{m}, C22^{m}), 46,6 (C14^{m}, C16^{m}), 46,0 (C14, C16), 33,1 (C34, C42), 30,8 (C35, C36, C47, C48), 30,7 (C37, C38, C49, C50), 30,6 (C39, C51), 30,5 (C40, C52), 27,3 (C41, C53), 27,2 (C42, C54), 23,8 (C43, C55), 14,5 (C44, C56). **Elementaranalyse:** ber.: C, 59,97; H, 8,90; N, 3,25; S, 7,45; gef.: C, 59,15; H, 8,92; N, 3,22; S, 7,13. **HRMS:** (ESI⁺, m/z) ber. für C₄₃H₇₇N₂O₁₁S₂ [M+H]⁺: 861,495738; gef.: 861,495738.

### Beispiel 3

### Herstellung von 1,5-Bis(3-methoxy-4-tetradecyloxyphenyl)-3-oxo-1,5-pentandisulfon-säure-diammoniumsalz (3)

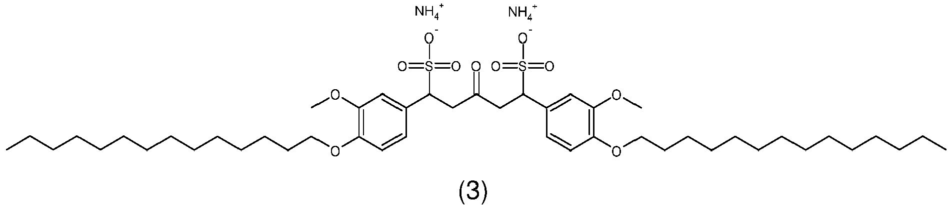

Stufe 1:
Die Reaktion erfolgte analog zu jener in Beispiel 1, außer dass 1-Bromtetradecan anstelle von 1-Bromoctan eingesetzt wurde, wobei 3-Methoxy-4-tetradecyloxybenzaldehyd als cremefarbenes Pulver erhalten wurde (Ausbeute: 12,1 g; 99,2 % d.Th.).

Stufe 2:
Die Reaktion erfolgte analog zu jener in Beispiel 1, außer dass das Rohprodukt nach dem Waschen mit MeOH aus siedendem Heptan umkristallisiert wurde, wobei 1,5-Bis(3-methoxy-4-tetradecyloxyphenyl)penta-1,4-dien-3-on als gelbes Pulver erhalten wurde (Ausbeute: 1,17 g; 46,4 % d.Th.).

Stufe 3:
Die Reaktion erfolgte analog zu jener in Beispiel 2, wobei die Titelverbindung (3) als cremefarbenes Pulver erhalten wurde (Ausbeute: 0,72 g; 78,3 % d.Th.).

**¹H-NMR:** δ_{H} (300 MHz, MeOD) 6,97 (1 H, s (C6, C12)), 6,88 (1 H, s (C6^{m}, C12^{m})), 6,79-6,74 (2 H, m (C3, C4, C8, C9)), 6,74-6,62 (2 H, m (C3^{m}, C4^{m}, C8^{m}, C9^{m})), 4,39-4,29 (1 H, m (C13, C18)), 4,28-4,20 (1 H, m (C13^{m}, C18^{m})), 4,02-3,89 (4 H, m, (C33, C47)), 3,81 (3 H, s (C20, C22)), 3,76 (3 H, s (C20^{m}, C22^{m})), 3,36-3,16 (4 H, m (C14, C16)), 1,87-1,72 (4 H, m (C34, C48)), 1,55-1,46 (4 H, m (C35, C49)), 1,42-1,25 (42 H, m (C36-C45, C50-C59)), 0,92 (6 H, t, J6,9 (C46, C60)). ¹³**C-NMR:** δ_{C} (75 MHz, MeOD) 207,4 (C15^{m}), 206,9 (C15), 150,2 (C1, C11), 150,1 (C1^{m}, C11^{m}), 149,4 (C2, C10), 149,3 (C2^{m}, C10^{m})), 130,4 (C5, C7), 130,3 (C5^{m}, C7^{m}), 123,0 (C4, C8), 122,4 (C4^{m}, C8^{m}), 114,8 (C6, C12), 114,0 (C3, C9), 113,8 (C3^{m}, C9^{m})), 70,2 (C33, C47), 70,1 (C33^{m}, C47^{m}), 62,6 (C13^{m}, C18^{m}), 62,3 (C13, C18), 56,4 (C20, C22), 56,3 (C20^{m}, C22^{m}), 46,6 (C14^{m}, C16^{m}), 46,0 (C14, C16), 33,1 (C34, C48), 31,0-30,3 (m) (C35-C42, C49-C56), 27,3 (C43, C57), 27,2 (C44, C58), 23,8 (C45, C59), 14,5 (C46, C60). **Elementaranalyse:** ber.: C, 61,54; H, 9,23; N, 3,05; S, 6,99; gef.: C, 59,58; H, 9,32; N, 2,93; S, 6,22. **HRMS:** (ESI⁻, m/z) ber. für C₄₇H₈₅N₂O₁₁S₂ [M+H]⁺: 917,558929; gef.: 917,557029.

### Beispiel 4

### Herstellung von 1,5-Bis(4-hexadecyloxy-3-methoxyphenyl)-3-oxo-1,5-pentandisulfonsäure-diammoniumsalz (4)

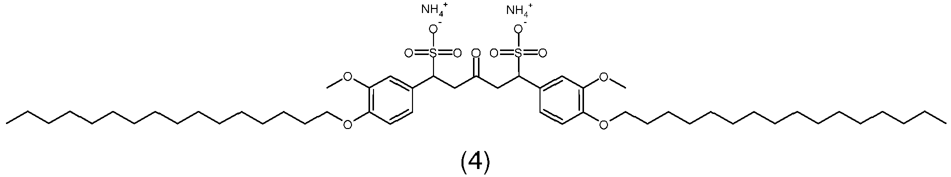

Stufe 1:
Die Reaktion erfolgte analog zu jener in Beispiel 1, außer dass 1-Bromhexadecan anstelle von 1-Bromoctan eingesetzt wurde, wobei 4-Hexadecyloxy-3-methoxybenzaldehyd als cremefarbenes Pulver erhalten wurde (Ausbeute: 13,0 g; 98,7 % d.Th.).

Stufen 2 & 3 (Eintopfreaktion):
4-Hexadecyloxy-3-methoxybenzaldehyd (2,63 g, 7 mmol) wurde in 14 ml Isopropanol bei 45 °C 12 h lang mit Aceton (0,20 g, 3,5 mmol) in Gegenwart von Lithiumhydroxid-monohydrat LiOH.H₂O (14,7 g, 0,35 mmol) als Katalysator umgesetzt. Anschließend wurden Triethylamin (0,07 g, 0,7 mmol), 10,5 ml einer 1 M wässrigen Lösung von Ammoniumhydrogensulfit, NH₄HSO₃ sowie weitere 38,5 ml Isopropanol zugesetzt, wonach das Reaktionsgemisch unter heftigem Rühren 14 h lang rückflusserhitzt wurde.
Anschließend wurde mittels eines mit Sauerstoffgas gefüllten Ballons das Reaktionsgemisch unter O₂-Atmosphäre gesetzt, um nichtumgesetztes Sulfit zu Sulfat zu oxidieren. Danach wurden 50 ml Pentan zugesetzt und das Wasser unter Verwendung eines Wasserabscheiders nach Dean-Stark bei 50 °C als Azeotrop abdestilliert, wonach der flüssige Rückstand am Rotationsverdampfer im Vakuum eingeengt wurde. Der so erhaltene Rückstand wurde mit Aceton versetzt und unter Verwendung eines Soxhlet-Extraktionsaufsatzes extrahiert. Anorganische Salze wurden durch Waschen mit MeOH entfernt, wonach von dem verbliebenen Rückstand das Lösungsmittel am Rotationsverdampfer entfernt und der Rückstand im Vakuumexsikkator vollständig getrocknet wurde, wodurch die Titelverbindung (4) als cremefarbenes Pulver erhalten wurde (Ausbeute: 2,17 g; 63,8 % d.Th.).

**¹H-NMR:** δ_{H} (300 MHz, MeOD) 6,94 (s, 1H, C6, C12), 6,85 (d, *J* = 1,7 Hz, 1H, (C6^{m}, C12^{m})), 6,73 (d, *J* = 1,1 Hz, 1H, (C3, C4, C8, C9)), 6,65 (d, *J* = 2,3 Hz, 3H, (C3^{m}, C4^{m}, C8^{m}, C9^{m})), 4,31 (dd, *J* = 9,3, 5,2 Hz, 1H, (C13, C18)), 4,22 (dd, *J* = 10,4, 4,0 Hz, 1H, (C13^{m}, C18^{m})), 3,94 (t, *J* = 6,6, 6,6 Hz, 4H (C33, C49)), 3,78 (s, 2H (C20, C22), 3,74 (s, 4H (C20^{m}, C22^{m})), 1,76 (s, 4H (C34, C50)), 1,29 (s, 44H(C35-C47, C51-C63), 0,89 (t, *J* = 6,8 Hz, 7H (C48, C64). **Elementaranalyse:** ber.: C, 62,93; H, 9,53; N, 2,88; S, 6,59; gef.: C, 58,93; H, 9,50; N, 2,77; S, 6,19.

### Beispiel 5

### Herstellung von 1,5-Bis(3-methoxy-4-octadecyloxyphenyl)-3-oxo-1,5-pentandisulfonsäure-diammoniumsalz (5)

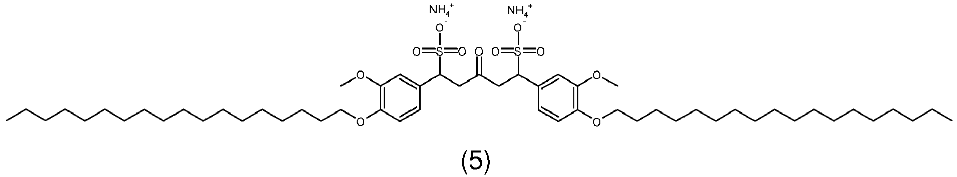

Stufe 1:
Die Reaktion erfolgte analog zu jener in Beispiel 1, außer dass 1-Bromoctadecan anstelle von 1-Bromoctan sowie das 10-fache Lösungsvolumen eingesetzt wurden, wobei 3-Methoxy-4-octadecyloxybenzaldehyd als cremefarbenes Pulver erhalten wurde (Ausbeute: 1,38 g; 97,6 % d.Th.).

Stufen 2 & 3 (Eintopfreaktion):
Die Reaktion erfolgte analog zu jener in Beispiel 4 unter Verwendung von 3-Methoxy-4-octadecyloxybenzaldehyd anstelle von 4-Hexadecyloxy-3-methoxybenzaldehyd, wodurch die Titelverbindung (5) als cremefarbenes Pulver erhalten wurde (Ausbeute: 11,9 g; 33,0 % d.Th.).

**¹H-NMR** (300 MHz, MeOD) δ 6,94 (s, 1H), 6,85 (d, *J* = 1,6 Hz, 1H), 6,75-6,70 (m, 2H), 6,65 (d, *J* = 2,5 Hz, 2H), 4,32 (dd, *J* = 9,3, 5,2 Hz, 1H), 4,22 (dd, *J* = 10,3, 4,1 Hz, 1H), 3,94 (t, *J* = 6,6, 6,6 Hz, 4H), 3,79 (s, 3H), 3,74 (s, 3H), 1,82-1,72 (m, 5H), 1,54-1,22 (m, 62H), 0,88 (t, *J* = 7,0 Hz, 6H).

### Beispiel 6

### Herstellung von 1,5-Bis(4-dodecyloxy-3-methoxyphenyl)-3-oxo-1,5-pentandisulfon-säure-dinatriumsalz (6)

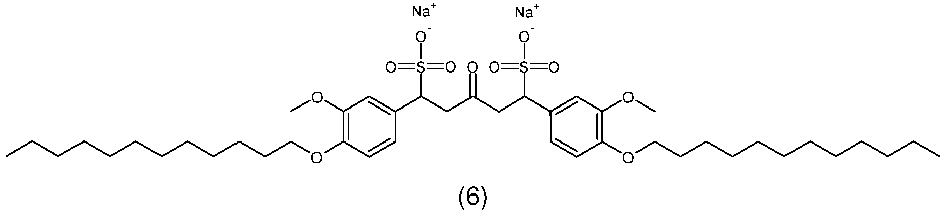

Stufe 1:
Die Synthese und das Produkt waren identisch mit Beispiel 2.

Stufen 2 & 3 (Eintopfreaktion):
Die Reaktion erfolgte analog zu jener in Beispiel 4, außer dass 4-Dodecyloxy-3-methoxy-benzaldehyd anstelle von 4-Hexadecyloxy-3-methoxybenzaldehyd und Natriumhydrogensulfit, NaHSO₃, anstelle von Ammoniumhydrogensulfit, NH₄HSO₃, als Sulfonierungsmittel eingesetzt wurden, wodurch die Titelverbindung (6) als cremefarbenes Pulver erhalten wurde (Ausbeute: 1,70 g; 55,7 % d.Th.).

**¹H-NMR** (300 MHz, MeOD) 6,95 (s, 1H), 6,85 (s, 1H), 6,77-6,71 (m, 2H), 6,65 (d, *J* = 2,0 Hz, 2H), 4,31 (dd, *J* = 8,6, 5,8 Hz, 1H), 4,22 (dd, *J* = 10,3, 4,1 Hz, 1H), 3,99-3,85 (m, 4H), 3,79 (s, 3H), 3,74 (s, 3H), 1,84-1,69 (m, 4H), 1,55-1,41 (m, 4H), 1,41-1,21 (m, 42H), 0,88 (t, *J* = 7,0 Hz, 6H).

### Beispiel 7

### Herstellung von 1,5-Bis(3-methoxy-4-tetradecyloxyphenyl)-3-oxo-1,5-pentandisulfon-säure-dinatriumsalz (7)

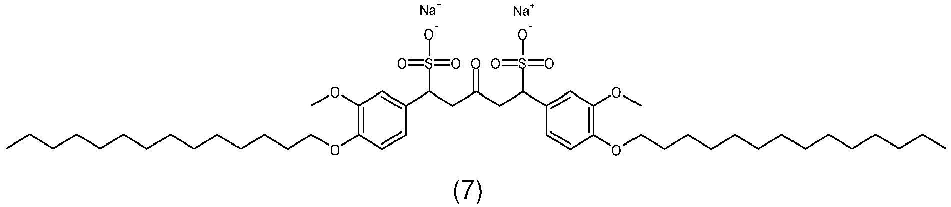

Stufe 1:
Die Synthese und das Produkt waren identisch mit Beispiel 3.

Stufen 2 & 3 (Eintopfreaktion):
Die Reaktion erfolgte analog zu jener in Beispiel 4, außer dass 3-Methoxy-4-tetradecyl-oxybenzaldehyd anstelle von 4-Hexadecyloxy-3-methoxybenzaldehyd und Natriumhydrogensulfit, NaHSO₃, anstelle von Ammoniumhydrogensulfit, NH₄HSO₃, als Sulfonierungsmittel eingesetzt wurden, wodurch die Titelverbindung (6) als cremefarbenes Pulver erhalten wurde (Ausbeute: 1,70 g; 55,7 % d.Th.).

**¹H NMR** (300 MHz, MeOD) δ 6,95 (s, 1H), 6,86 (d, *J* = 1,5 Hz, 2H), 6,74 (s, 2H), 6,66 (d, *J=* 1,8 Hz, 3H), 4,32 (dd, *J* = 9,6, 4,8 Hz, 1H), 4,24 (dd, *J* = 10,4, 3,9 Hz, 2H), 3,94 (d, *J* = 7,0 Hz, 4H), 3,79 (s, 3H), 3,74 (s, 4H), 3,39-3,33 (m, 1H), 3,28-3,14 (m, 2H), 1,84-1,69 (m, 4H), 1,56-1,42 (m, 4H), 1,41-1,20 (m, 44H), 0,91 (t, *J* = 6,3, 6,3 Hz, 6H). **HRMS:** (ESI⁻, m/z) ber. für C₄₇H₇₇N₂O₁₁S₂ [M+H]⁺: 927,469721; gef.: 927,469727.

### Beispiel 8

### Herstellung von 1,5-Bis(4-hexadecyloxy-3-methoxyphenyl)-3-oxo-1,5-pentandisulfonsäure-dinatriumsalz (8)

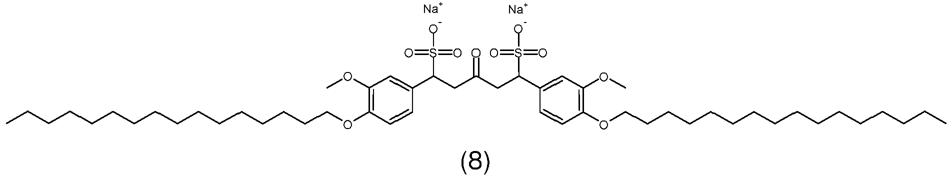

Stufe 1:
Die Synthese und das Produkt waren identisch mit Beispiel 4.

Stufen 2 & 3 (Eintopfreaktion):
Die Reaktion erfolgte analog zu jener in Beispiel 4, außer dass Natriumhydrogensulfit, NaHSO₃, anstelle von Ammoniumhydrogensulfit, NH₄HSO₃, als Sulfonierungsmittel eingesetzt wurde, wodurch die Titelverbindung (8) als cremefarbenes Pulver erhalten wurde (Ausbeute: 2,66 g; 77,3 % d.Th.).

**¹H-NMR:** δ_{H} (700 MHz, MeOD) δ 6,96 (s, 1H), 6,88 (d, *J=* 1,8 Hz, 1H), 6,75 (d, *J* = 1,2 Hz, 1H), 6,70-6,65 (m, 3H), 4,34 (dd, J = 10,3, 4,2 Hz, 1H), 4,25 (dd, *J* = 10,8, 3,7 Hz, 1H), 3,96 (t, *J* = 6,5 Hz, 4H), 3,80 (s, 2H), 3,76 (s, 4H), 3,36-3,33 (m, 1H), 3,31-3,20 (m, 2H), 1,78 (dt, *J* = 8,7, 6,7 Hz, 4H), 1,49 (td, *J* = 8,0, 7,6, 4,2 Hz, 4H), 1,42-1,36 (m, 5H), 1,35-1,28 (m, 49H), 0,91 (t, *J* = 7,0 Hz, 5H).**¹³C-NMR:** δ_{C} (75 MHz, MeOD) (176 MHz, MeOD) δ 207,5, 207,0, 150,2, 150,1, 149,4, 149,3, 130,4, 130,3, 123,1, 122,4, 114,8, 114,0, 113,9, 70,2, 70,1, 62,6, 62,3, 46,6, 46,0, 33,1 (d, *J* = 2,1 Hz), 30,9, 30,8, 30,8, 30,8, 30,8, 30,7, 30,6, 30,6, 30,5-30,4 (m), 27,3, 27,2, 23,8, 14,5. **HRMS:** (ESI⁺, m/z) ber. für C₅₁H₈₅Na₂O₁₁S₂ [M+H]⁺: 983,532321; gef.: 983,531847.

### Beispiel 9

### Herstellung von 1,5-Bis(3-methoxy-4-octadecyloxyphenyl)-3-oxo-1,5-pentandisulfonsäure-dinatriumsalz (9)

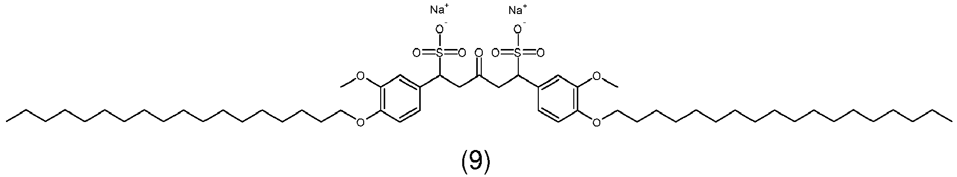

Stufe 1:
Die Synthese und das Produkt waren identisch mit Beispiel 5.

Stufen 2 & 3 (Eintopfreaktion):
Die Reaktion erfolgte analog zu jener in Beispiel 8, außer dass 3-Methoxy-4-octadecyl-oxybenzaldehyd anstelle von 4-Hexadecyloxy-3-methoxybenzaldehyd eingesetzt wurde, wodurch die Titelverbindung (9) als cremefarbenes Pulver erhalten wurde (Ausbeute: 1,05 g; 28,9 % d.Th.).

**¹H-NMR:** δ_{H} (300 MHz, MeOD) δ 6,96 (s, 1H), 6,87 (d, *J* = 1,6 Hz, 1H), 6,78-6,71 (m, 2H), 6,70-6,61 (m, 2H), 4,40-4,26 (m, 1H), 3,96 (dd, *J* = 7,6, 5,5 Hz, 4H), 3,80 (s, 2H), 3,76 (s, 3H), 1,84-1,70 (m, 5H), 1,30 (d, *J=* 3,2 Hz, 56H), 0,96-0,87 (m, 6H). **Elementaranalyse:** ber.: C, 63,55; H, 8,92; S, 6,17; gef.: C, 59,72; H, 9,06; S, 6,78. **HRMS:** (ESI⁺, m/z) ber. für C₅₅H₉₃Na₂O₁₁S₂ [M+H]⁺ 1039,594921; gef.: 1039,595834.

### Beispiel 10

### Herstellung von 1,5-Bis(4-hexadecyloxy-3-methoxyphenyl)-3-oxo-1,5-pentandisulfonsäure-dikaliumsalz (10)

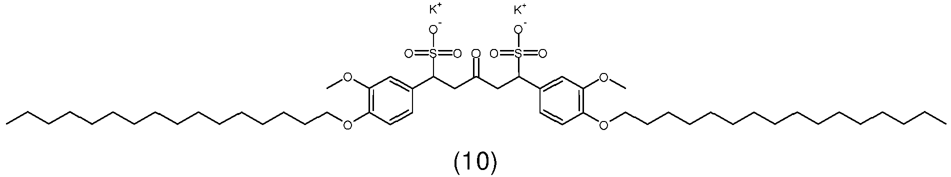

Stufe 1:
Die Synthese und das Produkt waren identisch mit Beispiel 4.

Stufen 2 & 3 (Eintopfreaktion):
Die Reaktion erfolgte analog zu jener in Beispiel 4, außer dass Kaliumhydrogensulfit, KHSO₃, anstelle von Ammoniumhydrogensulfit, NH₄HSO₃, als Sulfonierungsmittel eingesetzt wurde, wodurch die Titelverbindung (10) als cremefarbenes Pulver erhalten wurde (Ausbeute: 1,16 g; 32,8 % d.Th.).

**¹H-NMR:** δ_{H} (300 MHz, MeOD) 6,94 (s, 1H, C6, C12), 6,85 (d, *J* = 1,7 Hz, 1H, (C6^{m}, C12^{m})), 6,73 (d, *J* = 1,1 Hz, 1H, (C3, C4, C8, C9)), 6,65 (d, *J* = 2,3 Hz, 3H, (C3^{m}, C4^{m}, C8^{m}, C9^{m})), 4,31 (dd, *J* = 9,3, 5,2 Hz, 1H, (C13, C18)), 4,22 (dd, *J* = 10,4, 4,0 Hz, 1H, (C13^{m}, C18^{m})), 3,94 (t, *J* = 6,6, 6,6 Hz, 4H (C33, C49)), 3,78 (s, 2H (C20, C22), 3,74 (s, 4H (C20^{m}, C22^{m})), 1,76 (s, 4H (C34, C50)), 1,29 (s, 44H(C35-C47, C51-C63), 0,89 (t, *J* = 6,8 Hz, 7H (C48, C64). **Elementaranalyse:** ber.: C, 60,32; H, 8,34; K, 7,70; S, 6,31; gef.: C, 56,57; H, 8,62; S, 8,00. **HRMS:** (ESI⁺, m/z) ber. für C₅₁H₈₅K₂O₁₁S₂ [M+H]⁺: 1015,480195; gef.: 1015,480682.

### Beispiel 11

### Herstellung von 1,5-Bis(4-dodecyloxy-3-methoxyphenyl)-3-oxo-1,5-pentandisulfon-säure-dicholinsalz (11)

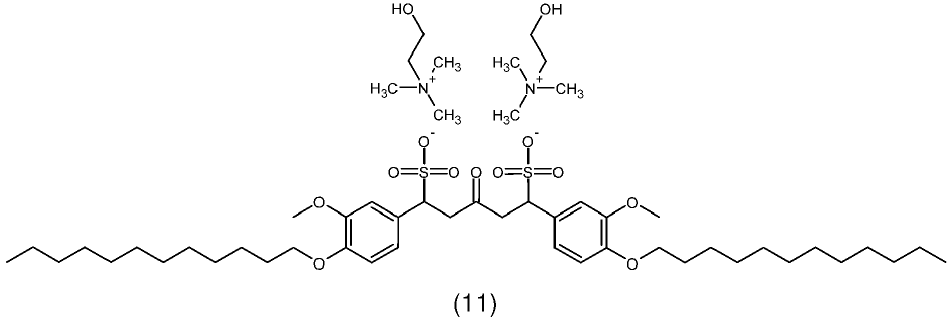

Stufe 1:
Die Synthese und das Produkt waren identisch mit Beispiel 2.

Stufe 2:
Die Synthese und das Produkt waren identisch mit Beispiel 2.

Stufe 3:
Die Reaktion erfolgte analog zu jener in Beispiel 1, Variante 3.1, außer dass nur 0,5 mmol 1,5-Bis(4-dodecyloxy-3-methoxyphenyl)penta-1,4-dien-3-on eingesetzt wurden und nach der Passage des Ionenaustauscherharzes eine wässrige Lösung von Cholinhydroxid anstelle der NH₄OH-Lösung in leichtem Überschuss zugesetzt wurde, wobei die Titelverbindung (11) als cremefarbenes Pulver erhalten wurde (Ausbeute: 0,52 g; 100,7 % d.Th.).

**¹H-NMR:** δ_{H} (300 MHz, MeOD) 6,95 (d, *J* = 1,2 Hz, 1H), 6,86 (d, *J* = 1,6 Hz, 1H), 6,75 (d, *J* = 1,1 Hz, 2H), 6,66 (d, *J* = 2,4 Hz, 2H), 4,31 (dd, *J* = 9,2, 5,1 Hz, 1H), 4,22 (dd, J = 10,3, 4,1 Hz, 1H), 4,01-3,89 (m, 8H), 3,79 (s, 3H), 3,74 (s, 3H), 3,48-3,39 (m, 4H), 3,16 (s, 20H), 1,82-1,69 (m, 4H), 1,29 (q, *J* = 5,2, 5,2, 4,2 Hz, 35H), 0,89 (d, *J* = 6,9 Hz, 6H). ¹³**C-NMR:** δ_{C} (75 MHz, MeOD) 207,4, 206,9, 150,2, 150,1, 149,4, 149,3, 130,6, 130,4, 123,1, 122,5, 114,8, 114,1, 113,9, 70,2, 70,1, 69,1-68,9 (m), 62,3, 57,1, 56,5, 56,4, 54,8-54,5 (m), 46,6, 46,0, 33,1, 31,0-30,4 (m), 27,3, 27,2, 23,8, 14,5. **Elementaranalyse:** ber.: C, 61,48; H, 9,54; N, 2,71; S, 6,19; gef.: C, 58,31; H, 9,56; N, 3,34; S, 6,02.

### Beispiel 12

### Herstellung von 1,5-Bis(4-dodecyloxy-3-methoxyphenyl)-3-oxo-1,5-pentandisulfonsäure-bis(triethanolammonium)salz (12)

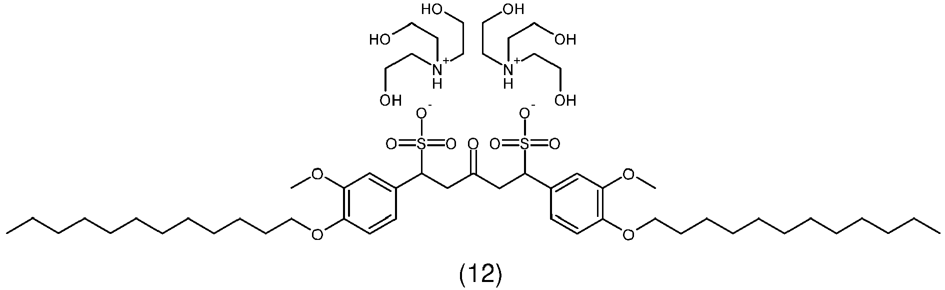

Stufe 1:
Die Synthese und das Produkt waren identisch mit Beispiel 2.

Stufe 2:
Die Synthese und das Produkt waren identisch mit Beispiel 2.

Stufe 3:
Die Reaktion erfolgte analog zu jener in Beispiel 1, Variante 3.1, außer dass nur 0,5 mmol 1,5-Bis(4-dodecyloxy-3-methoxyphenyl)penta-1,4-dien-3-on eingesetzt wurden und nach der Passage des Ionenaustauscherharzes eine wässrige Lösung von Triethanolamin anstelle der NH₄OH-Lösung in leichtem Überschuss zugesetzt wurde, wobei die Titelverbindung (12) als cremefarbenes Pulver erhalten wurde (Ausbeute: 0,55 g; 98,5 % d.Th.).

**¹H-NMR:** δ_{H} (300 MHz, MeOD) 7,0 (s, 1H), 6,9 (d, *J* = 1,6 Hz, 1H), 6,7 (d, *J* = 1,1 Hz, 2H), 6,7 (d, *J* = 2,1 Hz, 2H), 4,4-4,3 (m, 1H), 4,3-4,2 (m, 1H), 3,9 (t, *J* = 6,6, 6,6, 1,6 Hz, 4H), 3,9-3,8 (m, 14H), 3,8 (s, 3H), 3,7 (s, 3H), 3,4 (q, *J* = 5,2, 5,2, 5,0 Hz, 12H), 1,8-1,7 (m, 4H), 1,5-1,4 (m, 4H), 1,4-1,2 (m, 34H), 0,9-0,9 (m, 6H). ¹³**C-NMR:** δ_{C} (75 MHz, MeOD) 207,4, 206,9, 150,2, 150,1, 149,3, 130,5, 130,3, 123,1, 122,4, 114,8, 114,0, 113,8, 70,2, 70,1, 62,6, 56,9, 56,7, 56,4, 56,3, 46,6, 33,1, 30,8, 30,8, 30,7, 30,5, 27,3, 27,2, 23,8, 14,5. **Elementaranalyse:** ber.: C, 58,80; H, 8,79; N, 2,49; S, 5,71; gef.: C, 57,01; H, 9,21; N, 2,71; S, 5,29.

### Beispiel 13

### Herstellung von 1,1'-(2-Oxocyclopentan-1,3-diyl)-bis[(3-methoxy-4-octyloxyphenyl)-methansulfonsäure-ammoniumsalz] (13)

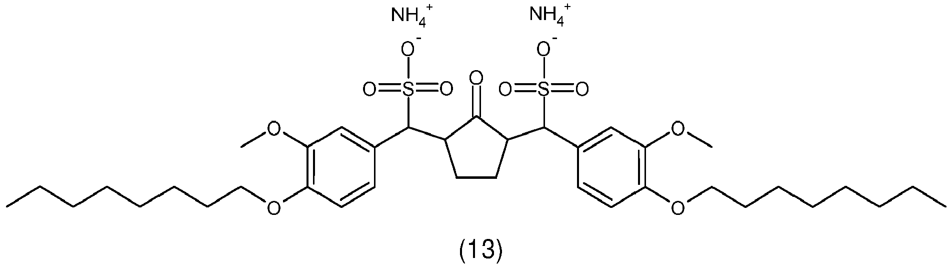

Stufe 1:
Die Synthese und das Produkt waren identisch mit Beispiel 1.

Stufe 2:
Die Reaktion erfolgte analog zu jener in Beispiel 1, außer dass der 4-Octyloxy-3-methoxybenzaldehyd mit Cyclopentanon (0,29 g, 3,5 mmol) anstelle von Aceton umgesetzt und der abzentrifugierte Rohprodukt-Niederschlag mit Hexan anstelle von MeOH gewaschen wurde, wobei 2,5-Bis(3-methoxy-4-octyloxybenzyliden)cyclopentan-1-on als gelbes Pulver erhalten wurde (Ausbeute: 1,78 g; 87,9 % d.Th.).

Stufe 3:
Die Reaktion erfolgte analog zu jener in Beispiel 1, Variante 3.1, außer dass 2,5-Bis(3-methoxy-4-octyloxybenzyliden)cyclopentan-1-on (0,29 g, 0,5 mmol) mit 6,2 ml einer 0,8 M wässrigen Lösung von schwefeliger Säure, H₂SO₃, in Gegenwart von Triethylamin (0,59 g, 10 mmol) umgesetzt wurde, wobei die Titelverbindung (13) in Form von gelblichen Kristallen erhalten wurde (Ausbeute: 0,37 g; 96,1 % d.Th.).

**¹H-NMR:** δ_{H} (300 MHz, MeOD) 7,19-7,03 (m, 2H), 6,98-6,74 (m, 3H), 6,48-6,32 (m, 1H), 4,63-4,37 (m, 2H), 4,07-3,84 (m, 5H), 3,83-3,67 (m, 6H), 3,39-3,31 (m, 2H), 3,23 (d, *J* = 7,9 Hz, 1H), 2,61 (d, *J* = 30,9 Hz, 1H), 2,53 (s, 1H), 2,35 (q, *J* = 11,3, 9,5 Hz, 1H), 1,91 (ddd, J = 11,7, 8,2, 3,7 Hz, 1H), 1,86-1,70 (m, 4H), 1,60-1,39 (m, 6H), 1,37-1,23 (m, 16H), 0,94-0,86 (m, 6H). ¹³**C-NMR:** δ_{C} (75 MHz, MeOD) 215,8, 148,8, 148,6, 147,9, 130,5, 127,3, 126,5, 122,9, 122,0, 114,4, 113,6, 113,3, 112,5, 111,8, 68,8, 68,7, 68,4, 64,7, 64,3, 63,9, 55,0, 54,8, 53,4, 51,4, 49,6, 47,3, 47,0, 46,7, 31,7, 31,6, 31,6, 29,3, 29,1, 29,0, 29,0, 29,0, 28,9, 25,9, 25,7, 23,3, 22,4, 22,3, 13,0. **Elementaranalyse:** ber.: C, 57,34; H, 8,06; N, 3,61; S, 8,27; gef.: C, 57,34; H, 8,50; N, 4,50; S, 6,85. **HRMS:** (ESI⁺, m/z) ber. für C₃₇H₆₃N₂O₁₁S₂ [M+H]⁺: 775,386779; gef.: 775,385622.

Eine exakte Zuordnung der Peaks in den NMR-Spektren war nicht möglich, da die Verbindung vier Stereozentren umfasst und das Produkt ein Gemisch von Diastereomeren darstellt.

### Beispiel 14

Tests bezüglich der Eignung der isolierten Amin-N-oxid-Verbindungen der Formel (I) und (II) als Tenside

Als Parameter für die Tensideigenschaften der neuen Sulfonat-Verbindungen wurde wie üblich die kritische Mizellenbildungskonzentration ("critical micelle concentration", CMC), d.h. die Konzentration, ab der sich Mizellen bilden können, mittels eines K100C Force Tensiometers von Krüss Scientific gemäß der Wilhelmy-Platten-Methode bei 25 °C und natürlichem pH bestimmt. Zum Vergleich wurde Dodecyldimethylaminsulfonat ("Vergleich") unter den gleichen Bedingungen vermessen. Die Ergebnisse sind in der nachstehenden Tabelle 1 angegeben, wobei niedrigere Werte eine stärkere Tensidwirkung der jeweiligen Substanz anzeigen.

**Tabelle 1**

| **Verbindung** | **CMC (mg/l)** |
|---|---|
| (1) | 3,2 × 10² |
| (2) | 64 |
| (3) | 34 |
| V1 | 6,8 × 10² |

Man erkennt, dass die drei getesteten erfindungsgemäßen Beispiele niedrigere ― und zwar mehrheitlich deutlich niedrigere ― CMC-Werte aufweisen als die in einer Vielzahl von im Handel erhältlichen Produkten eingesetzte Vergleichssubstanz. Allerdings beträgt auch der CMC-Wert von 0,32 g/l für das Disulfonat (1) aus Beispiel 1, das von den drei erfindungsgemäßen Verbindungen aufgrund des Octyl-Rests R¹ die geringste Anzahl an Kohlenstoffatomen in den Resten R¹ bis R⁵ enthält, dennoch weniger als die Hälfte des handelsüblichen Tensids des Vergleichsbeispiels.

Aufgrund der Analogien bzw. starken Ähnlichkeiten der Substitutionsmuster der übrigen, bisher noch nicht getesteten erfindungsgemäßen Verbindungen ist für den einschlägigen Fachmann zu erwarten, dass auch für diese durchwegs eine starke Tensidwirkung nachweisbar sein wird. Dies gilt gleichermaßen für die hierin auf Basis von Vanillin synthetisierten Substanzen (1) bis (13) wie für jegliche weitere Verbindungen, die unter Verwendung von ähnlichen Lignin-Abbauprodukten als Ausgangsmaterialien gemäß vorliegender Erfindung herstellbar sind, zumal diese mehrheitlich sogar eine größere Anzahl an Kohlenstoffatomen in den Resten R¹ bis R⁵ aufweisen, wie z.B. Syringaldehyd, das gegenüber Vanillin einen zusätzlichen Methoxy-Substituenten als Rest R³ umfasst.

Die vorliegende Erfindung stellt somit eine Gruppe von neuen Sulfonat-Verbindungen bereit, die mittels relativ einfacher Syntheseschritte auf wirtschaftliche und die Umwelt schonende Weise erhältlich sind und von denen sich die große Mehrzahl zur Verwendung als Tenside eignen.

## Patentansprüche

1. Sulfonat-Verbindung der Formel (I) oder (II): 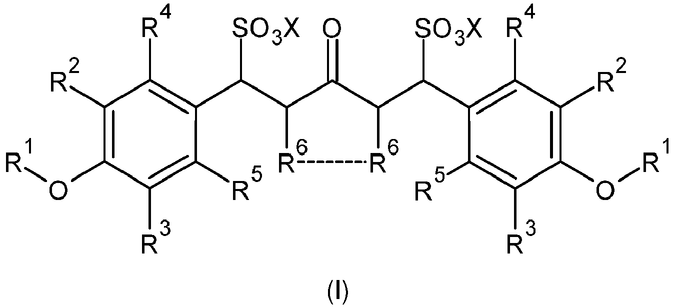 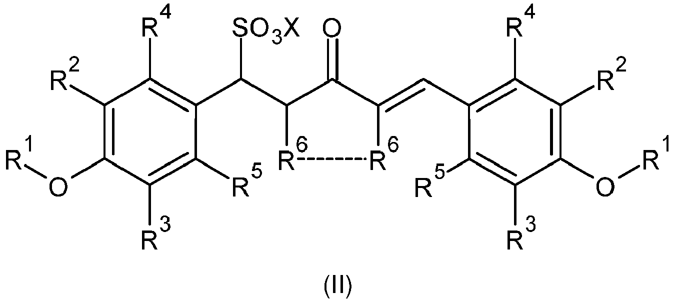 worin
die R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt sind;
R² bis R⁵ jeweils unabhängig aus Wasserstoff und linearen, verzweigten oder zyklischen Kohlenwasserstoff-Resten mit 1 bis 26 Kohlenstoffatomen, in denen gegebenenfalls zumindest ein Kohlenstoffatom durch ein Sauerstoff- oder Schwefelatom ersetzt ist, ausgewählt sind;
die R⁶ jeweils unabhängig aus Wasserstoff und gesättigten Kohlenwasserstoff-Resten mit 1 bis 6 Kohlenstoffatomen ausgewählt sind, wobei die beiden Reste R⁶ gegebenenfalls, wie durch die gestrichelte Linie angedeutet, miteinander zu einem den Carbonyl-Kohlenstoff umfassenden fünf- oder sechsgliedrigen Ring verbunden sind; und
die X jeweils unabhängig aus ein- und mehrwertigen Kationen Xⁿ⁺, worin n ≥ 1 ist, einschließlich H⁺, ausgewählt sind, wobei in Formel (I) gegebenenfalls beide X zusammen ein mehrwertiges Kation darstellen.

2. Sulfonat-Verbindung nach Anspruch 1, **dadurch gekennzeichnet, dass**
die R¹ jeweils für C₆-C₂₂-Alkyl stehen; und/oder
R² und R³ jeweils unabhängig aus Wasserstoff, C₁-C₂₂-Alkyl und C₁-C₂₂-Alkoxy ausgewählt sind; und/oder
R⁴ und R⁵ aus Wasserstoff, Methyl und Methoxy ausgewählt sind; und/oder die R⁶ aus Wasserstoff, Methyl und Ethyl ausgewählt sind; und/oder die X jeweils für H⁺, Na⁺, K⁺, NH₄⁺ oder ein organisches Ammoniumion stehen.

3. Sulfonat-Verbindung nach Anspruch 1 oder 2, **dadurch gekennzeichnet, dass**
die R¹ jeweils für C₈-C₁₈-Alkyl stehen; und/oder
R² und R³ jeweils unabhängig aus Wasserstoff, C₁-C₄-Alkyl und C₁-C₄-Alkoxy ausgewählt sind; und/oder
R⁴ und R⁵ jeweils Wasserstoff sind.

4. Sulfonat-Verbindung nach einem der Ansprüche 1 bis 3, **dadurch gekennzeichnet, dass**
die R¹ beide für denselben C₈-C₁₈-Alkylrest stehen;
einer von R² und R³ Wasserstoff ist und der andere Methoxy ist oder beide Reste Methoxy sind;
R⁴ und R⁵ jeweils Wasserstoff sind;
die R⁶ beide Wasserstoff oder Methyl sind oder miteinander zu einem Ethylen- oder Propylenrest verbunden sind und somit einen den Carbonyl-Kohlenstoff umfassenden fünf- oder sechsgliedrigen Ring bilden; und
die X jeweils für H⁺, Na⁺, NH₄⁺ oder ein organisches Ammoniumion stehen, das gegebenenfalls (2-Hydroxyethyl)trimethylammonium (Cholin) oder Triethanolammonium ist.

5. Sulfonat-Verbindung nach Anspruch 4, **dadurch gekennzeichnet, dass** sie aus den folgenden Verbindungen ausgewählt ist:
1,5-Bis(3-methoxy-4-octyloxyphenyl)-3-oxo-1,5-pentandisulfonsäure-diammoniumsalz (1) 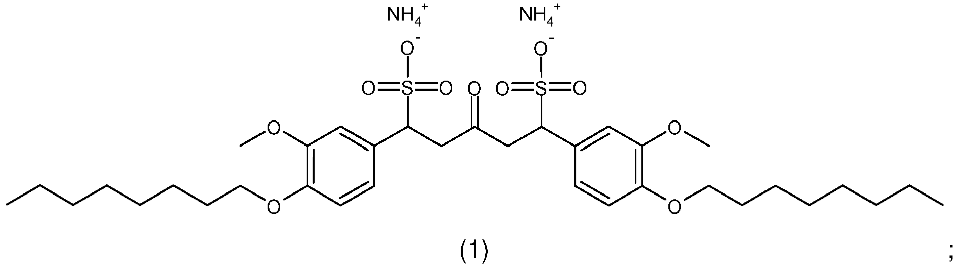
1,5-Bis(4-dodecyloxy-3-methoxyphenyl)-3-oxo-1,5-pentandisulfonsäure-diammoniumsalz (2) 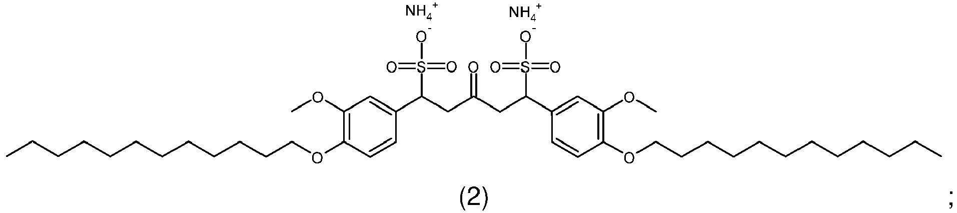
1,5-Bis(3-methoxy-4-tetradecyloxyphenyl)-3-oxo-1,5-pentandisulfonsäure-diammoniumsalz (3) 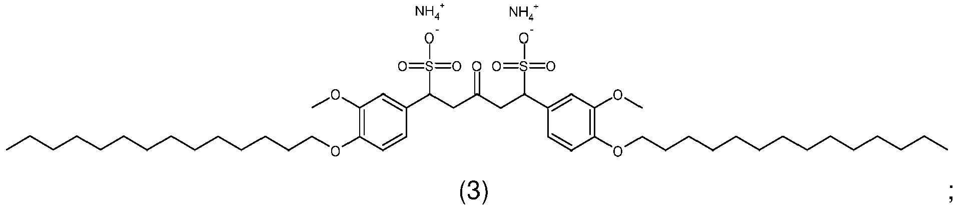
1,5-Bis(4-hexadecyloxy-3-methoxyphenyl)-3-oxo-1,5-pentandisulfonsäure-diammoniumsalz (4) 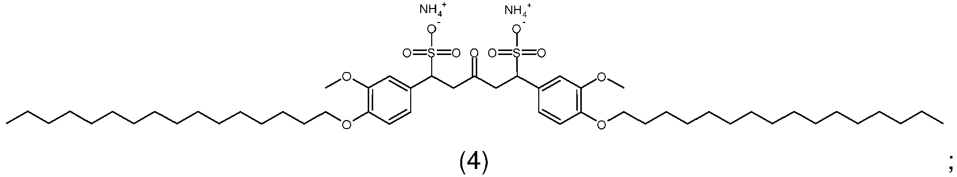
1,5-Bis(3-methoxy-4-octadecyloxyphenyl)-3-oxo-1,5-pentandisulfonsäure-diammoniumsalz (5) 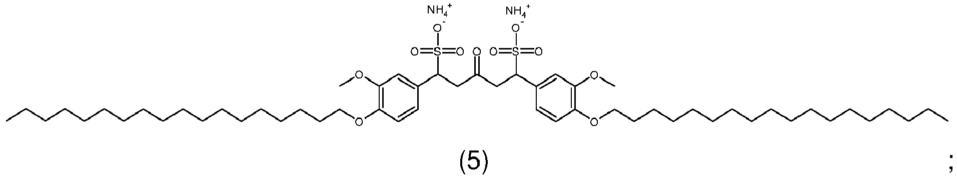
1,5-Bis(4-dodecyloxy-3-methoxyphenyl)-3-oxo-1,5-pentandisulfonsäure-dinatriumsalz (6) 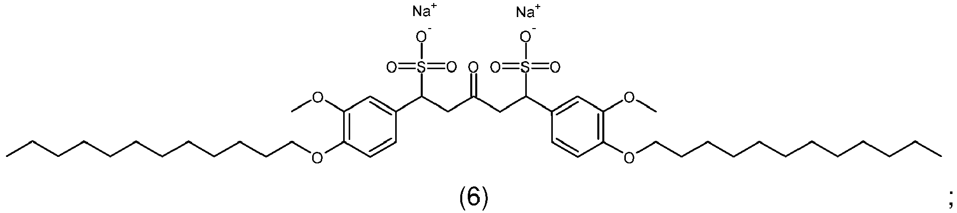
1,5-Bis(3-methoxy-4-tetradecyloxyphenyl)-3-oxo-1,5-pentandisulfonsäure-dinatriumsalz (7) 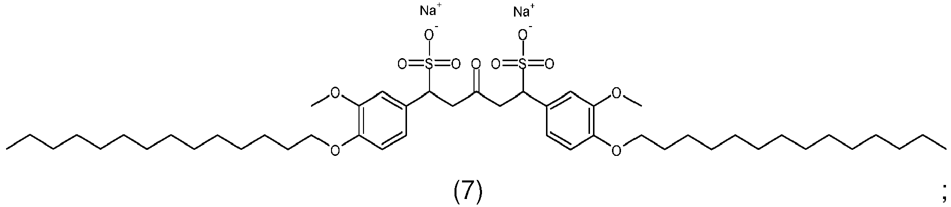
1,5-Bis(4-hexadecyloxy-3-methoxyphenyl)-3-oxo-1,5-pentandisulfonsäure-dinatriumsalz (8) 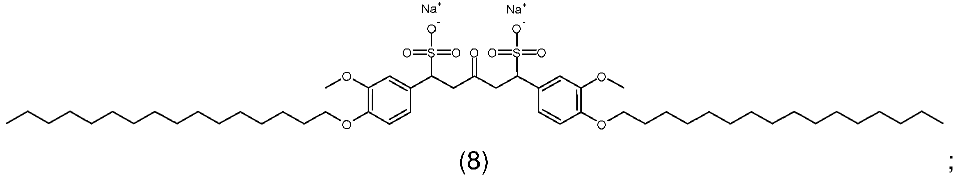
1,5-Bis(3-methoxy-4-octadecyloxyphenyl)-3-oxo-1,5-pentandisulfonsäure-dinatriumsalz (9) 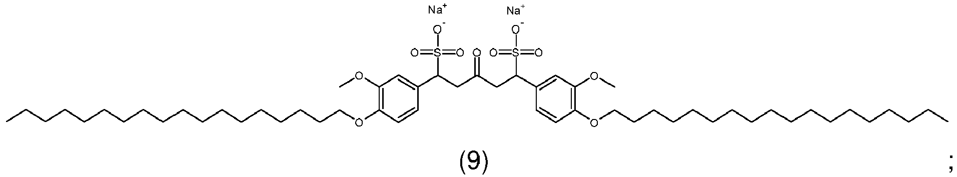
1,5-Bis(4-hexadecyloxy-3-methoxyphenyl)-3-oxo-1,5-pentandisulfonsäure-dikaliumsalz (10) 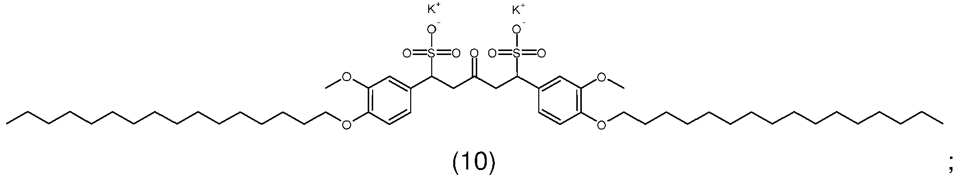
1,5-Bis(4-dodecyloxy-3-methoxyphenyl)-3-oxo-1,5-pentandisulfonsäure-dicholinsalz (11) 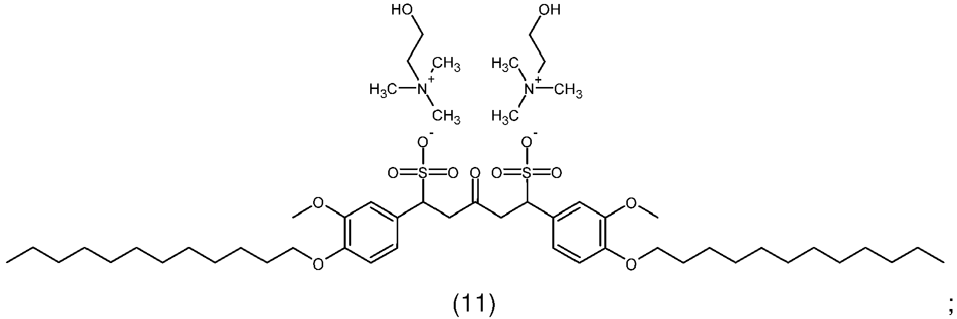
1,5-Bis(4-dodecyloxy-3-methoxyphenyl)-3-oxo-1,5-pentandisulfonsäure-bis(triethanolammonium)salz (12) 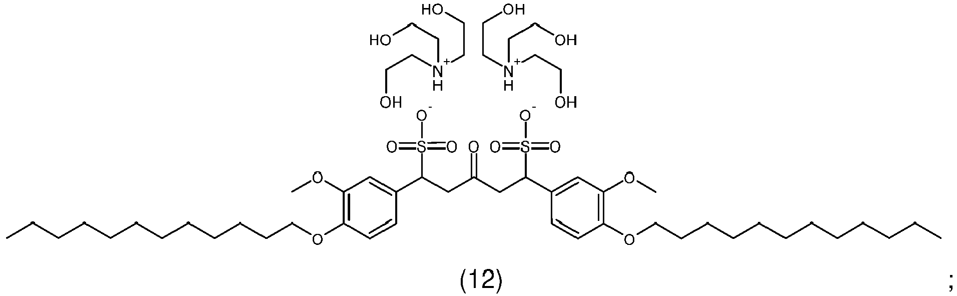
1,1'-(2-Oxocyclopentan-1,3-diyl)-bis[(3-methoxy-4-octyloxyphenyl)-methansulfonsäure-ammoniumsalz] (13)

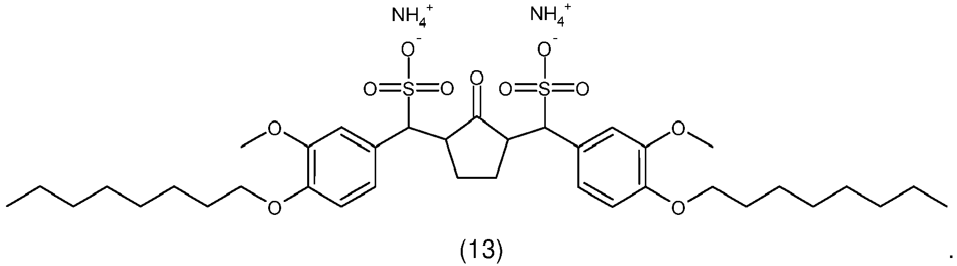

6. Verfahren zur Herstellung einer Sulfonat-Verbindung nach einem der Ansprüche 1 bis 5, das die folgenden Schritte umfasst:
1) das Umsetzen eines 4-Hydroxybenzaldehyd-Derivats der nachstehenden Formel (III): 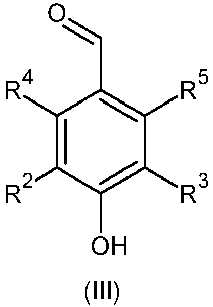 worin
R² bis R⁵ jeweils unabhängig aus Wasserstoff und linearen, verzweigten oder zyklischen Kohlenwasserstoff-Resten mit 1 bis 26 Kohlenstoffatomen, in denen gegebenenfalls zumindest ein Kohlenstoffatom durch ein Sauerstoff- oder Schwefelatom ersetzt ist, ausgewählt sind;
mit einer Verbindung der Formel R¹-Y, worin R¹ aus linearen, verzweigten oder zyklischen Kohlenwasserstoff-Resten mit 4 bis 26 Kohlenstoffatomen, in denen gegebenenfalls zumindest ein Kohlenstoffatom durch ein Sauerstoff- oder Schwefelatom ersetzt ist, ausgewählt ist und Y für eine aus Halogeniden und Sulfonaten ausgewählte Abgangsgruppe steht, mittels einer Veretherungsreaktion nach Williamson in Gegenwart einer Base in einem organischen Lösungsmittel, wodurch ein entsprechender Ether der Formel (IV) erhalten wird: 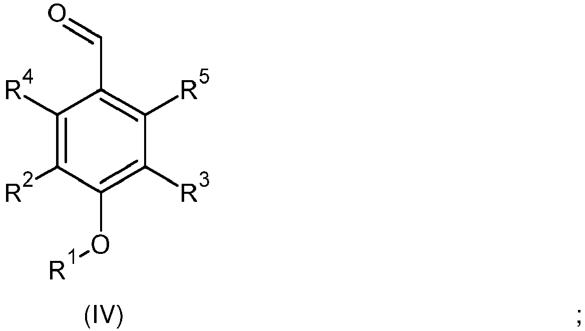
2) das Umsetzen des Ethers der Formel (IV) mit einem halben Äquivalent von Aceton oder einem Aceton-Derivat der Formel (V) 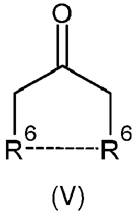 worin die R⁶ jeweils unabhängig aus Wasserstoff und gesättigten Kohlenwasserstoff-Resten mit 1 bis 6 Kohlenstoffatomen ausgewählt sind, wobei die beiden Reste R⁶ gegebenenfalls, wie durch die gestrichelte Linie angedeutet, miteinander zu einem den Carbonyl-Kohlenstoff umfassenden fünf- oder sechsgliedrigen Ring verbunden sind, mittels einer doppelten gekreuzten Aldol-Kondensationsreaktion nach Claisen-Schmidt unter Säure- oder Basenkatalyse in einem organischen Lösungsmittel, wodurch ein entsprechendes ungesättigtes Keton der Formel (VI) erhalten wird: 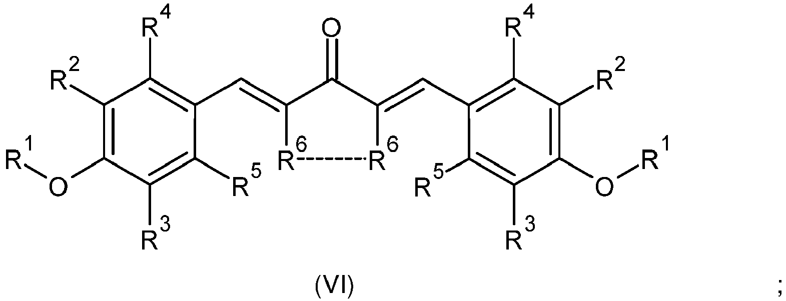
3) das Umsetzen des Ketons der Formel (VI) mit einem Sulfonierungsmittel in einem alkoholischen Lösungsmittel zur Addition von einem oder zwei Äquivalenten Hydrogensulfit an die Doppelbindung(en) des Ketons der Formel (VI), gegebenenfalls gefolgt von einem lonenaustausch des so erhaltenen Mono- oder Disulfonats sowie Einführung von vorbestimmten Gegenionen Xⁿ⁺, worin n ≥ 1 ist, um die Sulfonat-Verbindung der Formel (I) oder (II) zu erhalten.

7. Verfahren nach Anspruch 6, **dadurch gekennzeichnet, dass** in Schritt 1)
als Abgangsgruppe Y das Chlorid oder Bromid eingesetzt wird; und/oder
als Base K₂CO₃ eingesetzt wird; und/oder
Acetonitril als organisches Lösungsmittel eingesetzt wird.

8. Verfahren nach Anspruch 7, **dadurch gekennzeichnet, dass** in Schritt 1)
als Abgangsgruppe Y das Bromid eingesetzt wird,
zwei Äquivalente K₂CO₃ als Base eingesetzt werden, und
die Reaktion in Acetonitril unter Rückfluss durchgeführt wird.

9. Verfahren nach einem der Ansprüche 6 bis 8, **dadurch gekennzeichnet, dass** in Schritt 2)
Lithiumhydroxid-monohydrat LiOH.H₂O als basischer Katalysator eingesetzt wird; und/oder
als organisches Lösungsmittel ein niederer Alkohol, ein Ether oder ein Gemisch davon eingesetzt wird.

10. Verfahren nach Anspruch 9, **dadurch gekennzeichnet, dass** in Schritt 2)
Lithiumhydroxid-monohydrat LiOH.H₂O in einer Menge von 1-10 Mol-% als basischer Katalysator eingesetzt wird,
als organisches Lösungsmittel Isopropanol eingesetzt wird, und
die Reaktion bei 40-50 °C durchgeführt wird.

11. Verfahren nach einem der Ansprüche 6 bis 10, **dadurch gekennzeichnet, dass** in Schritt 3)
als Sulfonierungsmittel ein Hydrogensulfit oder Disulfit eingesetzt wird; und/oder
ein Gemisch aus einem niederen Alkohol und Wasser als alkoholisches Lösungsmittel eingesetzt wird; und/oder
ein Amin als Katalysator eingesetzt wird.

12. Verfahren nach Anspruch 11, **dadurch gekennzeichnet, dass** in Schritt 3)
als Sulfonierungsmittel Natriumhydrogensulfit NaHSO₃, Calciumhydrogensulfit Ca(HS0₃)₂, Ammoniumhydrogensulfit NH₄HSO₃ oder Trimethylammoniumsulfit [(CH₃)₃N]₂SO₃ eingesetzt wird,
Triethylamin, Triethanolamin oder Cholinhydroxid als Katalysator eingesetzt wird und
als alkoholisches Lösungsmittel wässriges Methanol oder Isopropanol eingesetzt wird.

13. Verfahren nach Anspruch 12, **dadurch gekennzeichnet, dass** in Schritt 3)
das Hydrogensulfit oder Disulfit in einer Menge von drei Äquivalenten Hydrogensulfit und der Amin-Katalysator in einer Menge von zumindest 20 Mol-%, jeweils bezogen auf das Keton der Formel (VI), eingesetzt werden, um eine Disulfonat-Verbindung der Formel (I) zu erhalten, und
als Lösungsmittel wässriges Isopropanol unter Rückfluss eingesetzt wird.

14. Verfahren nach einem der Ansprüche 6 bis 13, **dadurch gekennzeichnet, dass**
a) in Schritt 3) das erhaltene Sulfonat-Addukt zunächst einem lonenaustausch unter Verwendung eines sauren Ionenaustauscherharzes und Elution mit Wasser zur Überführung der Sulfonat-Gruppe(n) in (eine) freie Sulfonsäure-Gruppe(n) unterzogen wird, die gegebenenfalls mit einer wässrigen Lösung der Hydroxide vorbestimmter Gegenionen Xⁿ⁺ neutralisiert wird, um die Sulfonat-Verbindung der Formel (I) oder (II) zu erhalten; und/oder
b) die Schritte 2) und 3) als Eintopf-Reaktion durchgeführt werden.

15. Verwendung einer Sulfonat-Verbindung der Formel (I) nach einem der Ansprüche 1 bis 5 oder hergestellt nach einem der Ansprüche 6 bis 14, bei der die Gesamtanzahl der Kohlenstoffatome der Reste R¹ bis R⁵ zumindest 9 beträgt, als Tensid.
